# EUROPEAN PATENT APPLICATION

(11) **EP 1 716 857 A1**
(43) Date of publication of application: **02.11.2006**
(21) Application number: 05719577.8
(22) Date of filing: 21.02.2005
(51) Int. Cl.: A61K 31/7012, A61P 35/00, A61P 43/00

(54) **COMPOSITION FOR NKT CELL ACTIVATION**

(30) Priority: 19.02.2004 JP 2004043481
(71) Applicant: Kibun Food Chemifa Co., Ltd., Tokyo 1048553 (JP); Kumazawa, Yoshio, Kawasaki-shi, Kanagawa 2110035 (JP)
(72) Inventor: KUMAZAWA, Yoshio, 2110035 (JP); MURATA, Katsumi, KIBUN FOOD CHEMIFA CO., LTD., Tokyo 1048553 (JP); KAWAHARA, Kazuyoshi, 312, Mansion New Tabata, Tokyo 1140013 (JP); TAKIMOTO, Hiroaki, 1940013 (JP)
(74) Representative: Zimmermann, Gerd Heinrich
(86) International application number: PCT/JP2005/003234
(87) International publication number: WO 2005/079813

(57) **Abstract**

Provided is more effective composition for NKT cell activation.

A composition for NKT cell activation comprising a glycosphingolipid having a structure represented by the following formula (1): 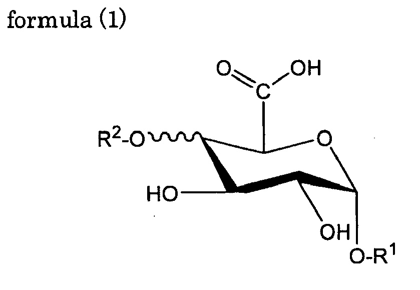 wherein R¹ represents the following formula (1-1): 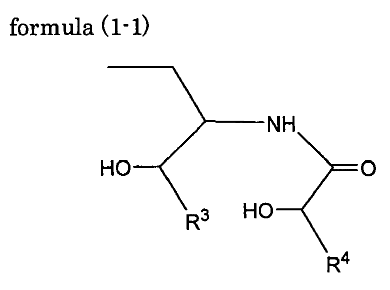 wherein R³ represents alkyl or alkenyl and R⁴ represents alkyl; and
R² represents hydrogen, or α-galactose, α-glucose, α-mannose, α-glucosamine, β-glucosamine or a combination thereof.

## Description

### Background art

The present invention relates to a composition for NKT cell activation, a composition for accelerating IL-4 production, a composition for accelerating IFN-γ production, a composition for dendritic cell activation, a composition for accelerating IL-12 production, a composition for accelerating IL-10 production, a composition for NK cell activation, an antitumor composition, an antiallergic composition, a composition for enhancing resistance to infection, an antiviral composition, a composition for accelerating IL-6 production, and a composition for accelerating NO production, comprising a glycosphingolipid with a given structure.

It has been heretofore considered that glycosphingolipids are present on the surface layers of animal cells or the like and that they are associated with the recognition mechanism. In contrast, gram-negative bacteria have outer membranes consisting of lipopolysaccharides, proteins, and phosphoric acids, on their cell cortexes, and they interact with the outer world via the outer membranes. Accordingly, lipopolysaccharides as the primary components of the outer membranes had been considered to be present in and to be essential for all gram-negative bacteria. In recent years, however, it has become known that the aerobic gram-negative bacteria *Sphingomonas paucimobilis*, previously known as "Pseudomonas paucimobilis," do not comprise lipopolysaccharides and comprise glycosphingolipids as bacterial lipids.

The present inventors have succeeded in isolating the glycolipids from the aforementioned *Sphingomonas paucimobilis*, analyzing the chemical structure thereof, and identifying the same (WO 92/12986). Also, the present inventors have disclosed that the aforementioned glycosphingolipids have excellent moisturizing effects and barrier effects and thus are extensively applicable as cosmetics (JP Patent Publication No. 11-43437). Further, the present inventors have elucidated the fact that the aforementioned glycosphingolipids have excellent emulsifying effects (JP Patent Publication No. 2000-51676).

The present inventors have also disclosed that glycosphingolipids obtained from bacterial strains of the other genus *Sphingomonas* are excellent as cosmetic and pharmaceutical compositions (JP Patent Publication No. 2002-010797).

It has been reported that the NKT cells expressing T cell receptors (TCR) are related to NK cells in the following respect: they exhibit large granular lymphocyte-like (LGL-like) in morphologies, they constantly express the IL-2R β chain, and they have perfolin. However, they are completely different from NK cells in terms of the possession of TCR (J. Immunol., 155, 2972, 1995).

Given the circumstances, it has been reported that mouse NKT cells that express NK1.1 in IL-12-activated T cells are important effector cells for inhibiting hematogenous metastasis of tumors to the liver or lung (J. Immunol., 154, 4333, 1995 and J. Immunol., 88, 82, 1996).

As described above, the NKT cells have drawn attention as a new group of cells in recent years.

Further, WO 98/44928 describes that α-glycosylceramide having a given structure is effective as an NKT cell activator. A more effective NKT cell activator has been desired.

### Disclosure of the Invention

An object of the present invention is to overcome the aforementioned problems and provide more effective for NKT cell activator. The object of the present invention is to provide a composition for accelerating IL-4 production, a composition for accelerating IFN-γ production, a composition for dendritic cell activation, a composition for accelerating IL-12 production, a composition for accelerating IL-10 production, a composition for NK cell activation, an antitumor composition, an antiallergic composition, a composition for enhancing resistance to infection, an antiviral composition, a composition for accelerating IL-6 production, and a composition for accelerating NO production, comprising glycosphingolipid.

Under the above circumstances, the present inventors found that the object could be attained by the following means.
(1) A composition for NK cell activation comprising a glycosphingolipid having a structure represented by the following formula (1): 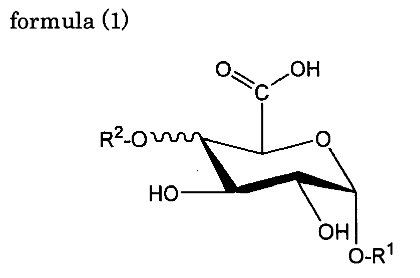 wherein R¹ represents the following formula (1-1): 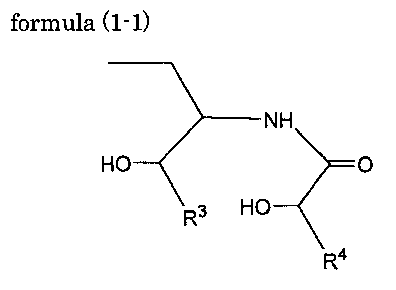 wherein R³ represents alkyl or alkenyl and R⁴ represents alkyl; and
   R² represents hydrogen, or α-galactose, α-glucose, α-mannose, α-glucosamine, β-glucosamine or a combination thereof.
(2)The composition for NKT cell activation according to (1), wherein the formula (1) is represented by the following formula (3): 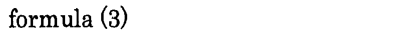 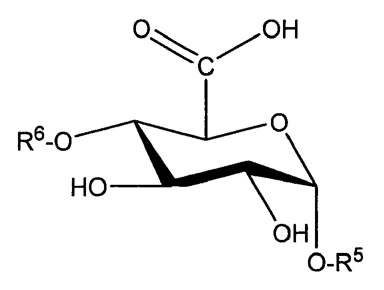 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵: 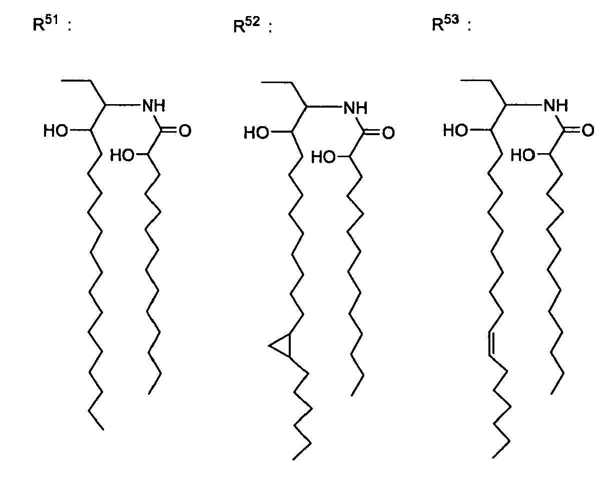 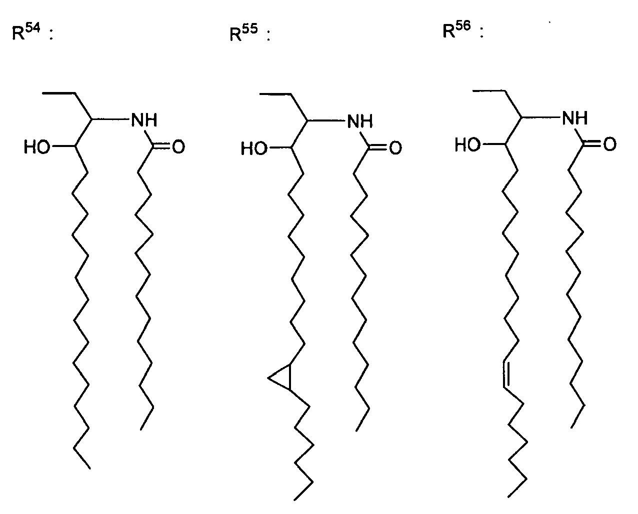 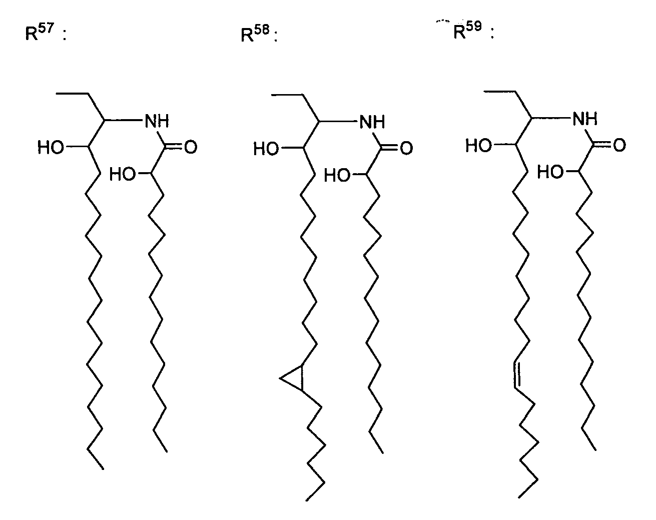 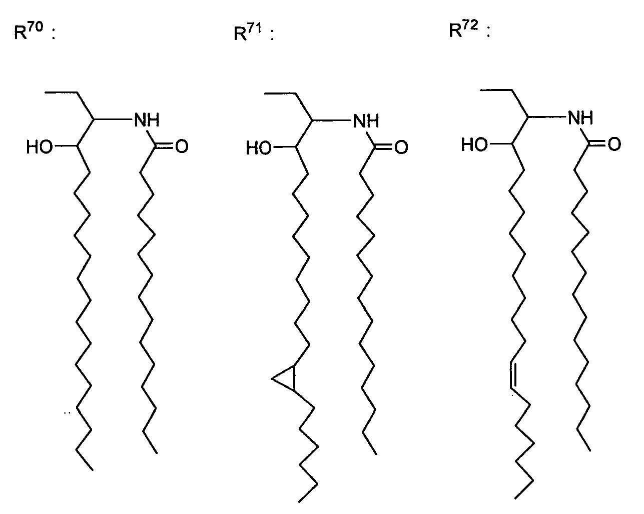 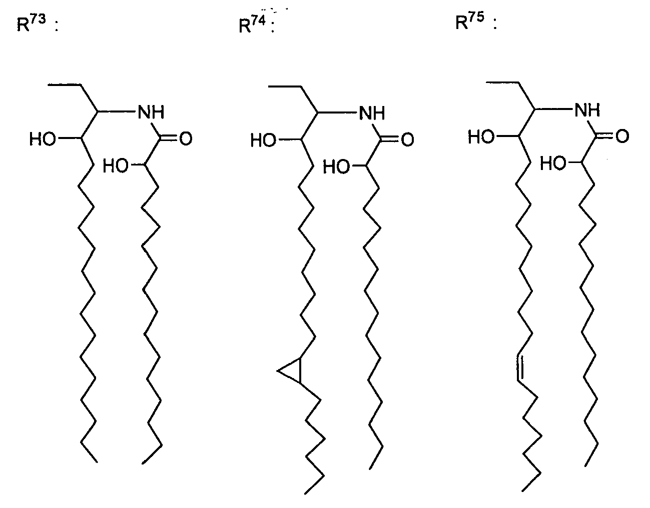 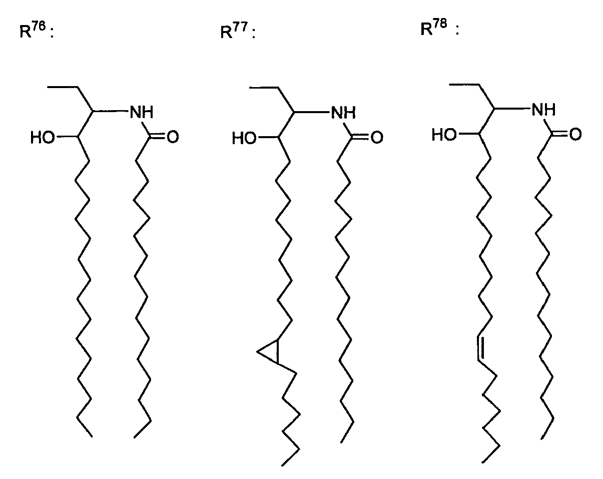 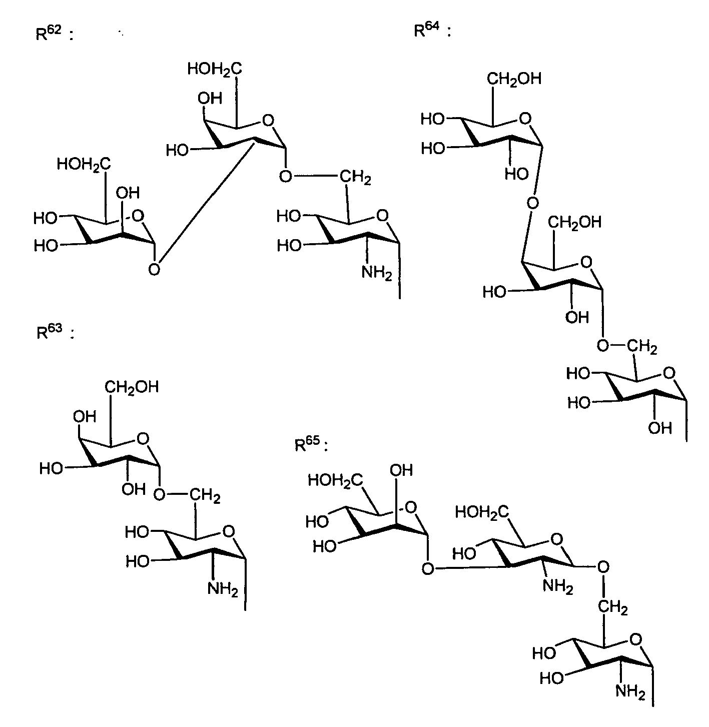
(3) A composition for accelerating IL-4 production comprising a glycosphingolipid having a structure represented by the following formula (1).
(4) The composition for accelerating IL-4 production according to (3), wherein the formula (1) is represented by the following formula (3).
(5) A composition for accelerating IFN-γ production comprising a glycosphingolipid having a structure represented by the following formula (1).
(6) The composition for accelerating IFN-γ production according to (5), wherein the formula (1) is represented by the following formula (3).
(7) A composition for dendritic cell activation comprising a glycosphingolipid having a structure represented by the following formula (1).
(8) The composition for dendritic cell activation according to (7), wherein the formula (1) is represented by the following formula (3).
(9) A composition for accelerating IL-12 production comprising a glycosphingolipid having a structure represented by the following formula (1).
(10) The composition for accelerating IL-12 production according to (9), wherein the formula (1) is represented by the following formula (3).
(11) A composition for accelerating IL-10 production comprising a glycosphingolipid having a structure represented by the following formula (1).
(12) The composition for accelerating IL-10 production according to (11), wherein the formula (1) is represented by the following formula (3).
(13) A composition for NK cell activation comprising a glycosphingolipid having a structure represented by the following formula (1).
(14) The composition for NK cell activation according to (13), wherein the formula (1) is represented by the following formula (3).
(15) An antitumor composition comprising a glycosphingolipid having a structure represented by the following formula (1).
(16) The antitumor composition according to (15), wherein the formula (1) is represented by the following formula (3).
(17) An antiallergic composition comprising a glycosphingolipid having a structure represented by the following formula (1).
(18) The antiallergic composition according to (17), wherein the formula (1) is represented by the following formula (3).
(19) A composition for enhancing resistance to infection comprising a glycosphingolipid having a structure represented by the following formula (1).
(20) The composition for enhancing resistance to infection according to (19), wherein the formula (1) is represented by the following formula (3).
(21) An antiviral composition comprising a glycosphingolipid having a structure represented by the following formula (1).
(22) The antiviral composition according to (21), wherein the formula (1) is represented by the following formula (3).
(23) A composition for accelerating IL-6 production comprising a glycosphingolipid having a structure represented by the following formula (1).
(24) The composition for accelerating IL-6 production according to (23), wherein the formula (1) is represented by the following formula (3).
(25) A composition for accelerating NO production comprising a glycosphingolipid having a structure represented by the following formula (1).
(26) The composition for accelerating NO production according to (25), wherein the formula (1) is represented by the following formula (3).

### Brief Description of the Drawings

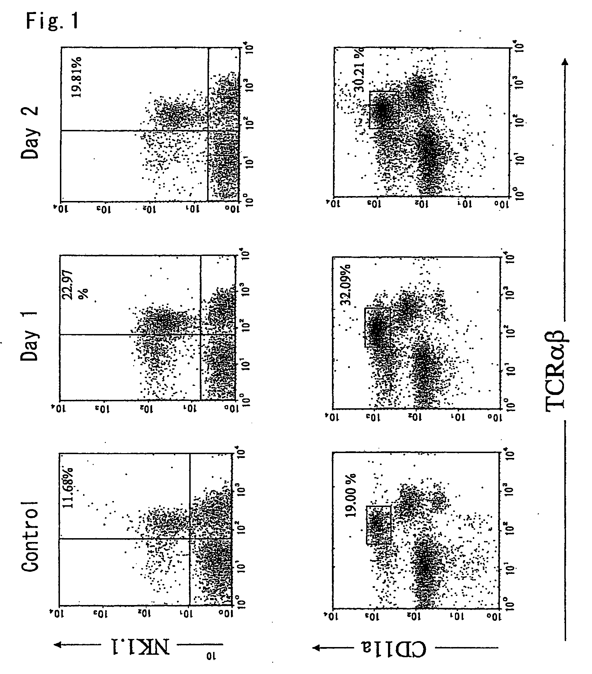

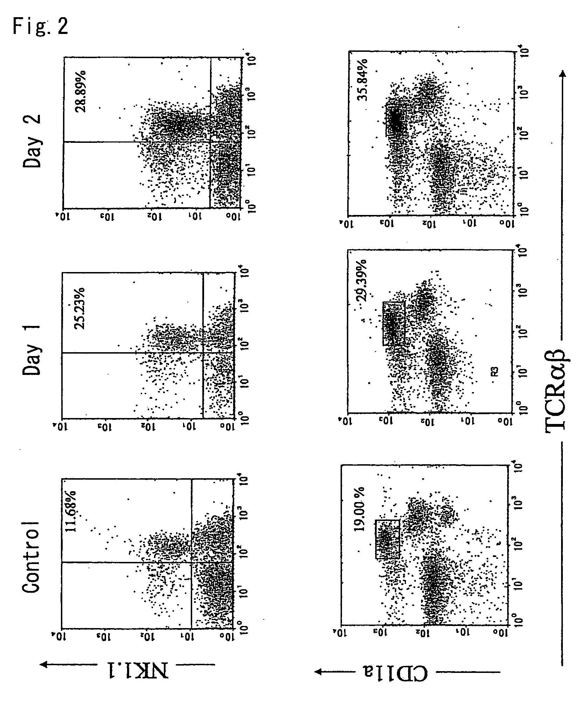

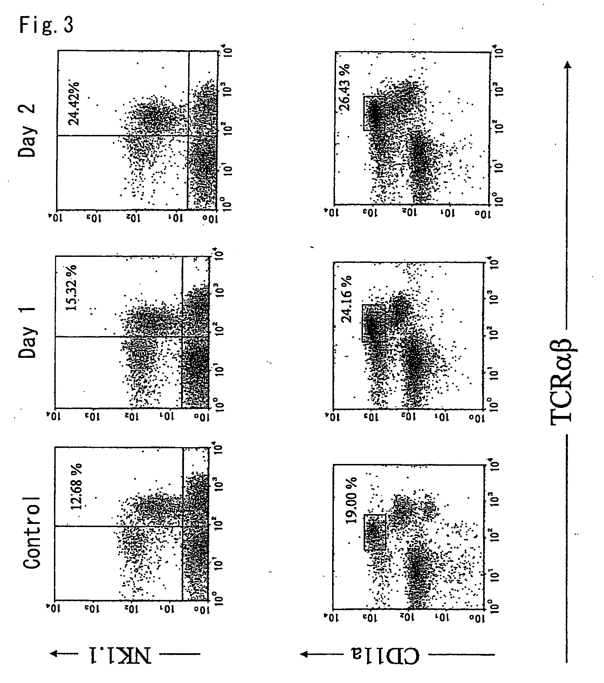

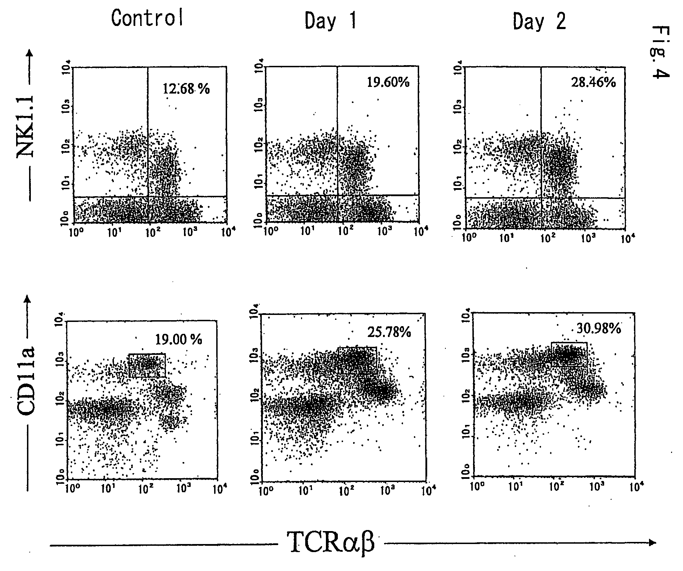

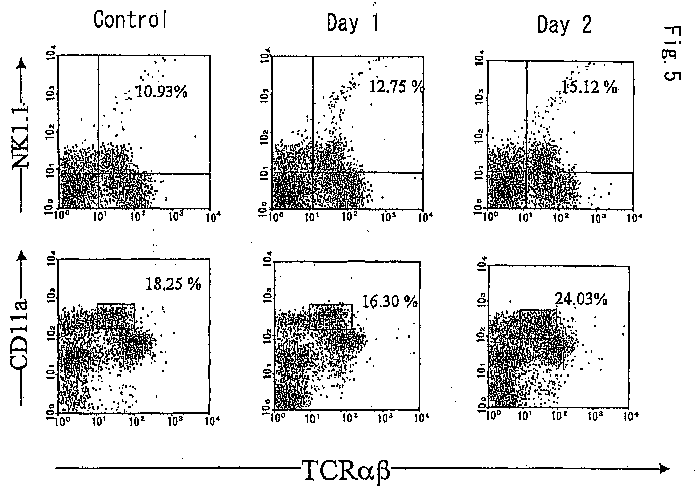

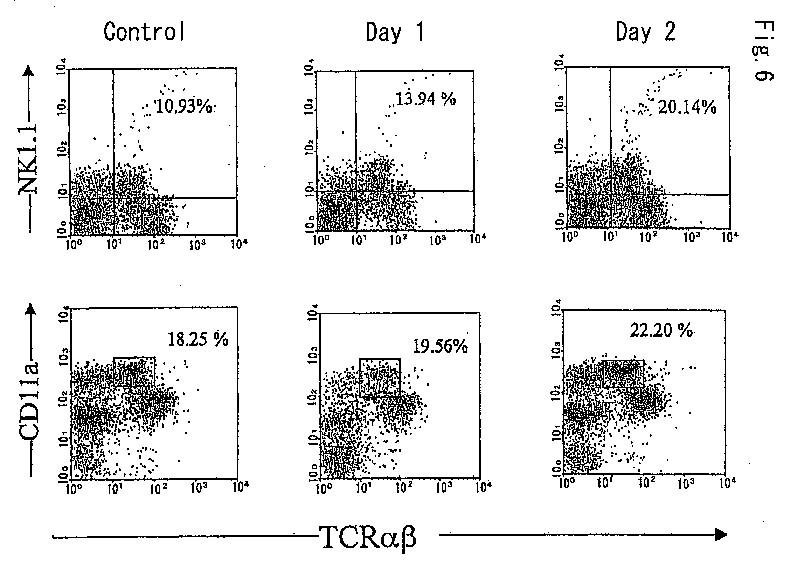

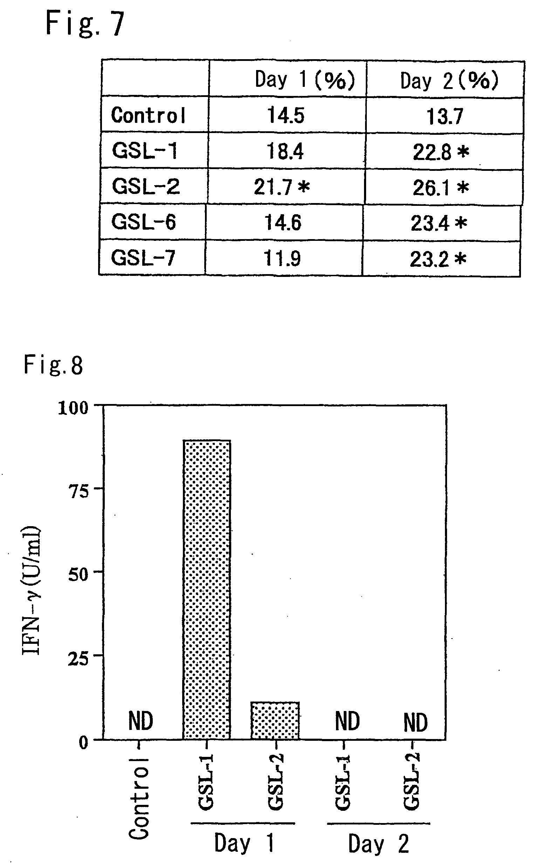

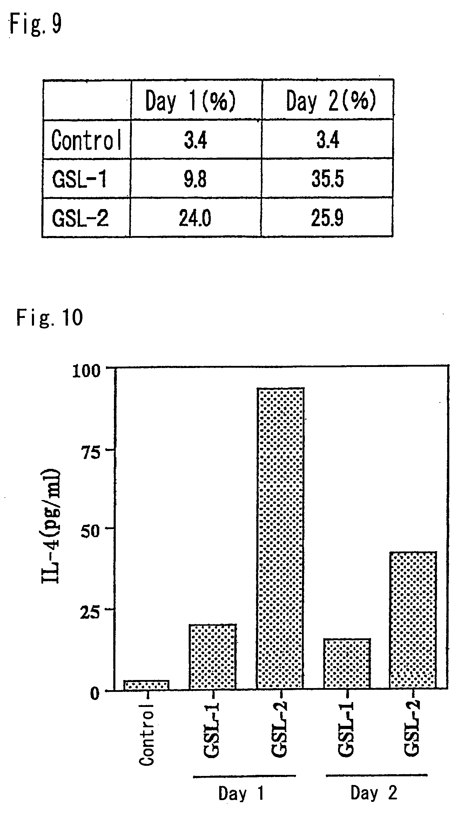

Fig. 1 shows the analysis results of flow cytometry when GSL-1 has been administered to a normal mouse. Fig. 2 shows the analysis results of flow cytometry when GSL-2 has been administered to a normal mouse. Fig. 3 shows the analysis results of flow cytometry when GSL-6 has been administered to a normal mouse. Fig. 4 shows the analysis results of flow cytometry when GSL-7 has been administered to a normal mouse. Fig. 5 shows the analysis results of flow cytometry when GSL-1 has been administered to a TLR4-deficient mouse. Fig. 6 shows the analysis results of flow cytometry when GSL-2 has been administered to a TLR4-deficient mouse. Fig. 7 shows NKT cell changes when GSL-1, 2, 6, or 7 has been administered to a normal mouse. Fig. 8 shows IFN-γ concentration after GSL-1 or GSL-2 has been administered to a normal mouse. Fig. 9 shows changes in IFN-γ-producing NKT cells when GSL-1 or GSL-2 has been administered to a normal mouse. Fig. 10 shows IL-4 concentration after GSL-1 or GSL-2 has been administered to a TLR4-deficient mouse.

### Detailed Description of the Invention

Hereafter, the present invention is described in detail. In the present specification, the symbol "~" is used to denote the numerical values that precede or follow the same as lower limits or upper limits. In this specification, R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, and R⁷⁸ in formula (3) may thus be represented as "R⁵¹ ~ R⁷⁸."

The glycosphingolipid that is used in the present invention (hereafter they may be referred to as "GSL(s)") have the structure represented by the formula (1). Alkyl represented by R³ that is included in R¹ in formula (1) may contain cycloalkyl, and the cycloalkyl may be present at the alkyl terminus or in the alkyl chain. A preferable example of cycloalkyl is cyclopropane. Alkyl represented by R³ has preferably 13 to 23, and more preferably 15, 16, 17, 18, 19, 20 or 21, carbon atoms. Alkyl or alkenyl represented by R³ preferably has a substituted or unsubstituted straight chain. A double bond may be present at any position in alkenyl.

In contrast, alkyl represented by R⁴ has preferably 10 to 20 carbon atoms, more preferably 10, 11, 12, 13, 14, 15 or 16 carbon atoms, and further preferably 10, 11, 12, 13, 14 or 15 carbon atoms. Alkyl represented by R⁴ preferably has a substituted or unsubstituted straight chain.

More preferably, R¹ is any of the aforementioned R⁵¹ ~ R⁷⁸.

R¹ in formula (1) preferably represents the conformation represented by the following formula (1-2): 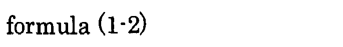 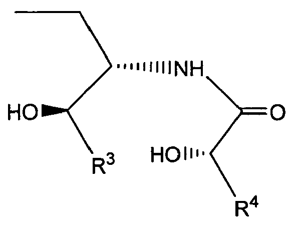 wherein R³ and R⁴ are as defined above in formula (1-1). Thus, the aforementioned R⁵¹ ~ R⁷⁸ having such conformations are more preferable.

Formula (1) is preferably represented by the formula (2), (3), (4), or (5). Formula (2) is as shown below: 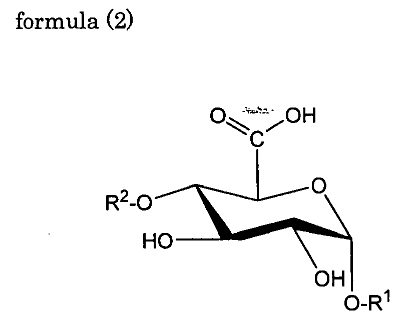 wherein R¹ and R² are as defined above in formula (1), and their preferable ranges are also as defined above.

In a preferable structure represented by the formula (3), R⁵ is any of R⁵¹ ~ R⁷⁸ and R⁶ is hydrogen (hereafter the same may be referred to as "Structure A"), R⁵ is any of R⁵¹ ~ R⁷⁸ and R⁶ is R⁶² (hereafter the same may be referred to as "Structure B"), R⁵ is any of R⁵¹ ~ R⁷⁸ and R⁶ is R⁶³ (hereafter the same may be referred to as "Structure C"), R⁵ is any of R⁵¹ ~ R⁷⁸ and R⁶ is R⁶⁴ (hereafter the same may be referred to as "Structure D"), or R⁵ is any of R⁵¹ ~ R⁷⁸ and R⁶ is R⁶⁵ (hereafter the same may be referred to as "Structure E"). Further, Structure A in which R⁵ is R⁵¹, R⁵², or R⁵³ is more preferably used.

Formula (4) is as shown below: 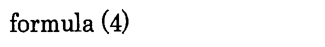 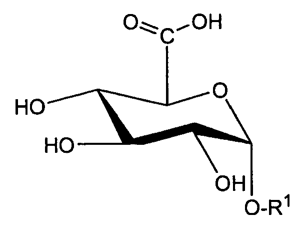 wherein R¹ is as defined above in formula (1), and its preferable range is also as defined above (a composition comprising at least one compound represented by the formula (4) may be hereafter referred to as "Structure AA").

Formula (5) is as shown below: 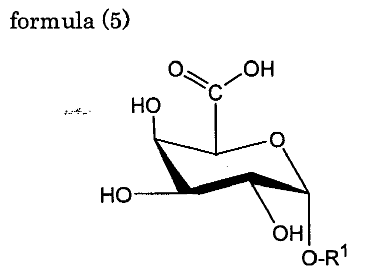 wherein R¹ is as defined above in formula (1) (hereafter the same may be referred to as "Structure F"), and its preferable range is also as defined above.

In the present invention, a single type or two or more types of glycosphingolipids may be used. When two or more types of glycosphingolipids are used in combination, the proportion of each component is not particularly limited. For example, a composition comprising at least one of the 3 types of compounds having Structure A, a composition comprising at least one of the 3 types of compounds having Structure B, or a composition comprising at least one of the compounds having Structure F may be used. Among them, a structure in which R¹ is any of R⁵¹, R⁵², or R⁵³ is preferable (hereafter the same may be referred to as "Structure FA").

In addition to the composition described above, the composition disclosed in the present invention may include composition represented by the formula (1) wherein formula (1-1) is represented by the conformation represented by the formula (1-1-1): 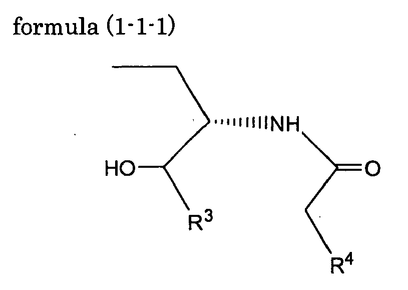 wherein R³ and R⁴ are as defined above in formula (1-1), and their preferable ranges are also as defined above.

Formula (1-1-1) further preferably has the structure represented by the formula (1-1-2): 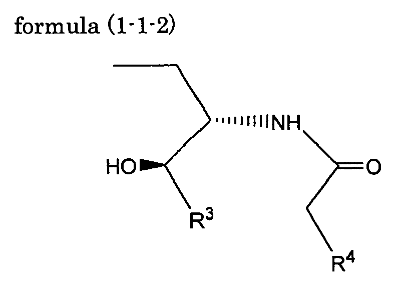 wherein R³ and R⁴ are as defined above in the formula (1-1), and their preferable ranges are also as defined above.

The glycosphingolipid represented by the formula (1) can be extracted from bacteria having glycosphingolipid. For example, methods disclosed in WO 92/12986 or JP Patent Publication No. 2002-10797 can be adopted. Since glycosphingolipid is contained in bacteria of the genus *Sphingomonas*, the glycosphingolipid represented by the formula (1) can be extracted from any of the bacteria of the genus *Sphingomonas.* The bacteria of the genus *Sphingomonas* include those that have been generally said to belong to the genus *Sphingomonas* and those that are classified as belonging to substantially the same genus as the genus *Sphingomonas.* For example, any bacteria described in Microbiol. Immunol., 2000, 44, 563-575 can be used in the present invention.

The glycosphingolipids represented by the formula (1) are insoluble in acetone. Accordingly, bacteria are preferably washed with acetone before extraction. An alcoholic solvent such as methanol or a mixed solvent comprising an alcoholic solvent mixed with a polar solvent such as chloroform is preferably used for extraction of the glycosphingolipid represented by the formula (1) from the viewpoint of yield. Other types of solvents may be used as long as such solvents are capable of dissolving glycosphingolipid.

When a mixture of glycosphingolipids is obtained, components thereof can be separated from one another in accordance with a technique known in the art. For example, glycosphingolipids can be completely separated via chromatography. When a mixed solution of chloroform and methanol is used as an eluate, glycosphingolipids of Structure A, Structure F, Structure C, and Structure B or Structure D or Structure E, are eluted in that order. Since Structure B, Structure D, and Structure E are generally produced by different bacteria, glycosphingolipids can be very easily separated. Conditions for separation via chromatography, such as a filler, an eluate, a rate of elution, pressure, and temperature, can be adequately regulated. Alternatively, a reagent that selectively reacts with a specific substance contained in the glycosphingolipid mixtures may be used in order to prepare a derivative of such substance, and separation can be carried out with the utilization of chemical or physical properties of such derivative. When the bacterium *Sphingomonas paucimobilis* is used, glycosphingolipids of Structure A and of Structure B are generally obtained. When the bacterium *Sphingomonas capsulata* (nomen novum: *Novosphingobium capsulatum*) is used, glycosphingolipids of Structure A and of Structure C are generally obtained. When the bacterium *Sphingomonas adhaesiva* is used, glycosphingolipids of Structure A and of Structure D are generally obtained. When *Sphingomonas* sp. MK346 is used, glycosphingolipids of Structure A and of Structure E are generally obtained. When *Sphingomonas wittichii, Sphingomonas macrogoltabidus* (nomen novum: *Sphingopyxis macrogoltabida*), *Sphingomonas terrae,* or *Sphingomonas yanoikuyae* (nomen novum: *Sphingobium yanoikuyae*) is used, glycosphingolipids of Structure AA (e.g., Structure A) and of Structure F (e.g., Structure FA) are generally obtained. Accordingly, glycosphingolipids of interest can be efficiently obtained by selecting bacteria based on such information.

The glycosphingolipid represented by the formula (1). can be also synthesized by combining conventional synthesis techniques. For example, a sugar and a sphingosine portion are first synthesized or extracted from the bacteria. Then, the glycosphingolipid represented by the formula (1) can be prepared by forming amide bonds between the sugar and the sphingosine portion.

In the present invention, NKT cells may include, for example, human Vα24⁺ NKT cells and mouse Vα14⁺ NKT cells. NKT cell activation may include enhancement of cytotoxic activity, enhancement of cytokine production, and acceleration of NKT cell proliferation. Further, the composition for NKT cell activation of the present invention accelerates the production of IL-4 and IFN-γ as a consequence. Accordingly, the composition disclosed in the present invention can be used as accelerators of various functions accelerated by IL-4 or IFN-γ. Among the GSLs that are used in the present invention, glycosphingolipid having galacturonic acid are particularly preferable as the composition for NKT cell activation.

Examples of functions accelerated by IL-4 may include Th2 induction and induction of antibody class switch. Examples of functions accelerated by IFN-γ may include Th1 induction and macrophage activation.

In addition to the aforementioned, the compositions for accelerating production or activation of each type of IL, IFN-α, IFN-β, tumor necrosis factors (TNF), lymphotoxin, hematopoietic colony-stimulating factors (CSF), erythropoietin, hematopoietic epidermal growth factors (EGF), and fibroblast growth factors (FGF) can be used as enhancement of cytokine. Further, the glycosphingolipids disclosed in the present invention can also be used as the other immune-activating composition, an apoptosis-inducing composition for cancer cells, or a composition for accelerating production or activation targeting acceleration of NF-kappaB activation, I-kappaB degradation, p38 phosphorylation, or Akt phosphorylation.

In the present invention, dendritic cell activation may include, for example, enhancement of antigen-presenting capacity.

GSLs according to the present invention accelerate production of IL-12 and of IL-10. Accordingly, such GSLs can also be used as a composition for accelerating various functions which are accelerated by the production of IL-12 and of IL-10. Glycosphingolipid containing glucuronic acids are particularly preferably used as the composition for accelerating IL-12 and/or IL-10 production of the present invention.

In the present invention, NK cell activation may include, for example, damage to infected cells. Among GSLs that are used in the present invention, monosaccharide type-glycosphingolipids are particularly preferably used as the composition for NK cell activation.

In the present invention, antitumor effects may include, for example, activation of helper T cells and of killer T cells. Examples of particularly preferable the antitumor composition among the GSLs used in the present invention may include tetrasaccharide-type glycosphingolipids.

In the present invention, antiallergic effect may include, for example, inhibition of eosinophilic infiltration, inhibition of mast cell activity, inhibition of IgE-production, and inhibition of mediator release.

The antiallergic composition of the present invention generally few side effects, and thus, application thereof for pollenosis, asthma bronchiale, endogenous eczema, or the like is particularly useful.

In the present invention, enhancing resistance to infection may include, for example, activation of helper T cells and of killer T cells. Application of the composition for enhancing resistance to infection according to the present invention for salmonella infection and tuberculosis is particularly useful. Among GSLs used in the present invention, tetrasaccharide type-glycosphingolipid is particularly preferable as the composition for enhancing resistance to infection.

In the present invention, antiviral effect includes inhibiting viral activity, for example, enhancement of type-I interferon production, activation of helper T cells, and activation of killer T cells. Application of the antiviral composition according to the present invention for Herpesviridae infections such as cytomegalovirus infection or herpesvirus infection is particularly useful.

When the composition of the present invention is used as pharmaceutical preparations, quasi-drugs, or active ingredients thereof, the composition can be preferably administered as a pharmaceutical composition that can be produced by a method known in the art. Examples of pharmaceutical composition may include tablets, capsules, powders, subtle granules, granules, liquids, and syrups. Such pharmaceutical compositions can be produced with the addition of pharmacologically or pharmaceutically acceptable additives. Examples of pharmacologically or pharmaceutically acceptable additives include vehicles, disintegrators or disintegration assistants, binders, lubricants, coating agents, dyes, diluents, bases, solubilizers or disintegration assistants, isotonizing agents, pH regulators, stabilizers, propellants, and adhesives. The pharmaceutical composition may also comprise one or more ingredients having other effects without departing from the scope of this invention. The dose of the pharmaceutical preparations according to the present invention is not particularly limited, and it can be adequately determined in accordance with the type of active ingredient or other conditions. Such dose can be adequately increased or decreased in accordance with a variety of general factors such as the body weight or age of the patient, the type or symptom of the disease, or the route of administration. In general, the pharmaceutical composition can be administered in amounts of 0.001 mg to 100 mg, and preferably 0.01 mg to 10 mg, per adult per day. The route of administration is not particularly limited, and administration can be made intravenously via injection or infusion or orally.

### Examples

The present invention is hereafter described in greater detail with reference to the examples. The materials, the amounts used, the percentages, the procedures, the processes, and other conditions described in the following examples can be adequately altered without departing from the scope of this invention. Accordingly, the technical scope of the present invention is not limited to the

### examples.

Structure A produced from *Sphingomonas paucimobilis* (GSL-1), Structure B produced from *Sphingomonas paucimobilis* (GSL-2), Structure C produced from *Sphingomonas capsulata* (GSL-3), Structure D produced from *Sphingomonas adhaesiva* (GSL-4), Structure E produced from *Sphingomonas* sp. MK346 (GSL-5), Structure AA produced from *Sphingomonas yanoikuyae* (GSL-6), Structure F produced from *Sphingomonas yanoikuyae* (GSL-7), Structure AA produced from *Sphingomonas wittichii* (GSL-8), Structure F produced from *Sphingomonas wittichii* (GSL-9), Structure AA produced from *Sphingomonas macrogoltabidus* (GSL-10), Structure F produced from *Sphingomonas macrogoltabidus* (GSL-11), Structure AA produced from *Sphingomonas terrae* (GSL-12), and Structure F produced from *Sphingomonas terrae* (GSL-13) were used in the examples. The GSLs were separated in accordance with the methods disclosed in WO 92/12986 and in JP Patent Publication No. 2002-010797.

### Example 1: Assay of activity of NKT cell activation

### Animals:

C57BL/10ScSn mice (hereafter referred to as "normal mice") and C57BL/10ScCr mice (TLR4-deficient/IL-12Rβ2 chain mutant mice) (hereafter referred to as "TLR4-deficient mice;" 7-week-old, female, the Max-Planck Institute of Immunobiology, Freiburg, Germany) were used as test animals. GSL-1 and GSL-2 activate macrophages of normal mice via TLR4 and induce IL-12 production. TLR4-deficient mice have mutation in the β-chain of the IL-12 receptor and thus IL-12 does not act. IL-12 is a substance that potently activates the immune system. With the use of the TLR4-deficient mice of the present example, acceleration of NKT cell activation by the glycosphingolipid that are used in the present invention can be further clarified without the influence of IL-12 activity.

### Preparation of samples:

The GSLs (10 µg) was administered to normal mice and to TLR4-deficient mice. Administration was carried out via caudal veins. Then, the serum and the liver were extracted from the mice, one of which had been administered physiological saline as a control (the control), another of which is one day after the administration (day 1), and the other of which is two days after the administration (day 2).

### Separation of intrahepatic leukocytes:

The sampled liver was crushed using 2 glass slides. A cell suspension was centrifuged at 500 rpm for 1 minute. The obtained supernatant was centrifuged at 1,200 rpm (300 g) for 5 minutes. The resulting sediment was suspended in 30% Percoll (Pharmacia), and the resulting suspension was superposed on 67.5% Percoll, centrifugation was carried out at 20°C at 2,000 rpm (800 g) for 30 minutes, and leukocytes were accumulated at the boundary of 30% Percoll and 67.5% Percoll and then collected. The obtained cells were washed three times with the Hanks medium to obtain intrahepatic leukocytes.

### Flow cytometry:

In order to block nonspecific binding of a fluorescently labeled antibody through FcR, anti-FcγR (2.4G2) was used. The PE-labeled anti-NK1.1 antibodies (PK136, Nippon Becton Dickinson Co., Ltd.) and the biotin-labeled anti-TCRαβ antibodies (H57-597, Nippon Becton Dickinson Co., Ltd.) were used. After the antibodies were added to the cells, the reaction was allowed to proceed at 4°C in the dark for 30 minutes. The cells treated with the biotin-labeled antibodies were allowed to react with Cy-chrome-bound streptavidin (554062, Nippon Becton Dickinson Co., Ltd.) at 4°C in the dark for 30 minutes. Staining of the aforementioned antibodies and Cy-chrome-bound streptavidin and washing of cells were carried out with the use of 0.1% NaN₃-containing 1% serum albumin. The cells were stained and then immobilized with 1% paraformaldehyde-containing phosphate buffered saline(PBS(-)). Assay was carried out using an automated cell sorter (Coulter Epics Elite ESP, Beckman Coulter). This procedure was separately carried out using the FITC-labeled anti-CD11a antibodies (M17/4, Nippon Becton Dickinson Co., Ltd.) instead of the PE-labeled anti-NK1.1 antibodies.

The flow cytometry was carried out by the method using the PE-labeled anti-NK1.1 antibodies (or CD11a). The results thereof are shown in Figs 1 to 6. Figs. 1 to 4 sequentially show the cases where GSL-1, GSL-2, GSL-6, and GSL-7 were administered to normal mice. Figs. 5 and 6 sequentially show the cases where GSL-1 and GSL-2 were administered to TLR4-deficient mice.

In the drawings, numerical values represent the percentages (%) of cells wherein both NK1.1 (or CD11a) and TCRαβ were expressed. It should be noted that the cells wherein both NK1.1 (or CD11a) and TCRαβ were expressed are NKT cells.

The flow cytometry for normal mice was carried out in the same manner as described above by the method using the PE-labeled anti-NK1.1 antibodies on a different day. The results thereof (the percentages of NKT cells) are shown in Fig. 7. In the drawing, numerical values represent the percentages (%) of cells wherein both NK1.1 (or CD11a) and TCRαβ were expressed, and the reference marks represent the cells that were found to be statistically significantly different from the control as a result of the t-test.

### Confirmation of the presence of IFN-γ:

The IFN-γ content in the blood after GSL-1 or GSL-2 had been administered was examined. GSL-1 or GSL-2 was administered to the normal mice and to the TLR4-deficient mice in the same manner as described above, and the IFN-γ content in the serum was analyzed.

The IFN-γ content was analyzed by the sandwich ELISA method using the purified anti-IFN-γ antibodies (R4-6A2, Nippon Becton Dickinson Co., Ltd.) and the biotin-conjugated anti-IFN-γ antibodies (AN-18, detection antibodies, Nippon Becton Dickinson Co., Ltd.) as a detectable antibody. After the reaction using the biotin-conjugated antibodies, alkaline phosphatase-labeled streptavidin (43-4822, Zymed) was conjugated thereto, and the color was developed using paranitrophenyl phosphate (N-4645, Sigma) as a substrate. The absorbance at 405 nm and that at 540 nm as the control were measured using a microplate reader (Model: 550, Nippon BioRad Laboratories). As a result, IFN-γ production was found to be accelerated in the normal mice, as shown in Fig. 8. Even though GSL-1 or GSL-2 was administered to the TLR4-deficient mice, IFN-γ was not detected because they did not react with IL-12.

### Flow cytometry of IFN-γ-producing NKT cells:

The normal mice to which GSL-1 or GSL-2 had been administered were subjected to flow cytometry in the same manner as described above, cells were stained with the PE-labeled anti-NK1.1 antibodies, the biotin-labeled anti-TCRαβ antibodies, and the FITC-labeled anti-IFN-γ antibodies (554410, Nippon Becton Dickinson Co., Ltd.), and the percentage of IFN-γ-producing NKT cells was determined. The results are shown in Fig. 9. In the drawing, numerical values represent the percentages of IFN-γ-producing NKT cells wherein both NK1.1 and TCRαβ were expressed.

### Measurement of IL-4 content:

Whether or not the IL-4 content in the blood increased after GSL-1 or GSL-2 had been administered was examined. GSL-1 or GSL-2 was administered to the normal mice and to the TLR4-deficient mice in the same manner as described above, and the IL-4 content in the serum was analyzed.

The IL-4 content was measured by ELISA using the BD Opti-EIA mouse IL-4 set (555232, Nippon Becton Dickinson Co., Ltd.) in accordance with the instruction manual. As a result, increase of IL-4 was observed in every mouse. In particular, IL-4 production was significantly accelerated in the TLR4-deficient mice, as shown in Fig. 10.

When GSL-1 or GSL-2 was added, increases in the percentages of cells wherein both NK1.1 and TCRαβ had been expressed were observed via the aforementioned flow cytometry. In the cells with significant increase in such expression levels, production of both IFN-γ and IL-4 was confirmed. This indicates that GSL-1 or GSL-2 is effective for activation of NKT cells.

### Example 2: Acceleration of IFN-γ production

The C57BL/6 mice were used as test mice. Each type of GSL was dissolved in 20% pentanediol to a final concentration of 10 mg/ml, the resulting solution was then dissolved in a solution diluted with physiological saline (hereafter the same may be referred to as "P") or a solution containing 0.5% pentanediol, 0.5% N-lauroylsarcosine, and 9.8% sucrose to a final concentration of 5 mg/ml, and the GSL was then treated with a solution diluted with physiological saline (hereafter the same may be referred to as "P+S"). GSL was administered to mice via the caudal veins (dose: 100 µg). The solvent alone was administered to mice as the control(control). A cell suspension was prepared from the liver extracted 16 hours after the administration, and intrahepatic leukocytes were obtained via specific gravity centrifugation using 45% Percoll and 67.5% Percoll. The leukocytes were treated with the anti-FcγR antibodies (2.4G2) and then treated with the FITC-labeled anti-IFN-γ antibodies, the PE-labeled anti-NK1.1 antibodies, and the biotin-labeled anti-TCRαβ antibodies. After the antibodies were added to the leukocytes, the reaction was allowed to proceed at 4°C in the dark for 30 minutes. The leukocytes treated with the biotin-labeled antibodies were allowed to react with the Cy-chrome-bound streptavidin at 4°C in the dark for 30 minutes. Staining with the aforementioned antibodies and Cy-chrome-bound streptavidin and washing of cells were carried out with the use of 0.1% NaN₃-containing 1% serum albumin. The cells were stained and then immobilized with 1% paraformaldehyde-containing PBS(-). Assay was carried out using the Epics Elite ESP as mentioned above. The results are shown in Table 1. In Table 1, the percentages (%) of the IFN-γ-producing NKT cells are shown. This experiment was separately carried out three times, i.e., experiment 1, experiment 2, and experiment 3.

**Table 1**

| | Sample | Percentage (%) of IFN-γ-producing NKT cells (mean ± S.D.) |
|---|---|---|
| Experiment 1 | Control | 2.0 ± 0.2 |
| | GSL-1 | 3.6 ± 0.3 |
| | GSL-2 | 2.6 ± 0.3 |
| | GSL-6 | 4.3 ± 1.4 |
| | GSL-7 | 14.0 ± 1.4 |
| Experiment 2 | Control | 3.7 ± 0.5 |
| | GSL-3 | 4.6 ± 0.6 |
| | GSL-4 | 5.2 ± 1.0 |
| | GSL-5 | 4.2 ± 0.3 |
| Experiment 3 | Control | 2.5 ± 0.5 |
| | GSL-8 | 4.7 ± 0.8 |
| | GSL-9 | 5.4 ± 1.2 |
| | GSL-10 | 3.8 ± 0.4 |
| | GSL-11 | 5.7 ± 0.5 |
| | GSL-12 | 3.2 ± 0.2 |
| | GSL-13 | 7.3 ± 3.0 |

As is apparent from Table 1, effects of accelerating IFN-γ production were observed. GSL-1, 2, 4, 6 to 11, and 13 were found to be more effective, GSL-6 to 9, 11, and 13 were found to be further effective, and GSL-7 was found to be remarkably effective.

### Example 3: Effects of dendritic cell activation

Effects of dendritic cell activation were examined. The C57BL/6 mice (7-week-old, female) were used. Each type of GSL was dissolved in the same manner as in Example 2. The samples were administered to mice through the caudal veins (dose: 100 µg). A cell suspension was prepared from the spleen extracted 12 hours after the administration. The leukocytes were treated with the anti-FcγR antibodies (2.4G2) and then treated with the PE-labeled anti-CD11c antibodies (Nippon Becton Dickinson Co., Ltd.), the biotin-labeled anti-CD40 antibodies (Nippon Becton Dickinson Co., Ltd.), the biotin-labeled anti-CD80 antibodies (Nippon Becton Dickinson Co., Ltd.), and the biotin-labeled anti-CD86 antibodies (Nippon Becton Dickinson Co., Ltd.). After the antibodies were added to the cells, the reaction was allowed to proceed at 4°C in the dark for 30 minutes. The cells treated with the biotin-labeled antibodies were allowed to react with Cy-chrome-bound streptavidin at 4°C in the dark for 30 minutes. Staining with the antibodies or Cy-chrome-bound streptavidin and washing of cells were carried out with the use of 0.1% NaN₃-containing 1% serum albumin. The cells were stained and then immobilized with 1% paraformaldehyde-containing PBS(-). Assay was carried out using the Epics Elite ESP. The percentages (%) of the CD40, CD80, and CD86 positive cells in the CD11c positive cells are shown in Table 2.

**Table 2**

| Sample | CD40 (%) (mean ± S.D.) | CD80 (%) (mean ± S.D.) | CD86 (%) (mean ± S.D.) |
|---|---|---|---|
| Control | 51.2 ± 0.6 | 72.8 ± 1.3 | 26.3 ± 1.8 |
| GSL-1 | 67.8 ± 1.9 | 81.9 ± 1.2 | 45.5 ± 0.9 |
| GSL-2 | 65.0 ± 9.8 | 76.2 ± 1.8 | 31.5 ± 1.7 |
| GSL-3 | 58.1 ± 2.9 | 79.9 ± 1.3 | 35.4 ± 2.7 |
| GSL-4 | 63.3 ± 0.1 | 80.6 ± 2.6 | 35.0 ± 0.2 |
| GSL-5 | 59.8 ± 0.7 | 77.4 ± 1.1 | 42.7 ± 17.7 |
| GSL-6 | 65.4 ± 2.5 | 81.0 ± 0.4 | 28.7 ± 3.0 |
| GSL-7 | 78.1 ± 3.5 | 85.4 ± 1.7 | 61.3 ± 22.5 |
| GSL-8 | 73.8 ± 4.2 | 83.9 ± 1.0 | 56.6 ± 24.6 |
| GSL-9 | 76.4 ± 1.1 | 84.9 ± 1.3 | 48.7 ± 6.1 |
| GSL-10 | 64.3 ± 4.6 | 78.9 ± 0.4 | 55.6 ± 30.4 |
| GSL-11 | 71.9 ± 2.4 | 80.9 ± 1.2 | 55.4 ± 19.8 |
| GSL-12 | 64.1 ± 2.1 | 81.3 ± 2.0 | 34.2 ± 51.2 |
| GSL-13 | 77.3 ± 0.8 | 85.2 ± 1.3 | 46.5 ± 1.7 |

As is apparent from Table 2, effects of dendritic cell activation were observed. GSL-1, 2, 4, and 6 to 13 were found to be more effective, and GSL-7 to 9, 11, and 13 were found to be remarkably effective.

### Example 4: Induction of IL-12 and induction of IL-10

Bone marrow cells of normal mice that had been used in Example 1 were cultured in the presence of GM-CSF and IL-4 for 8 days to obtain dendritic cells derived from bone marrow cells. Each type of GSL shown in the table below was dissolved in an ethanol:dodecane (98:2) solvent at 0.5 mg/ml, 20 µl each thereof was dispensed into each well of a 96-well plate, the solvent was vaporized, and the GSL was immobilized on the plate. As the control, the ethanol:dodecane (98:2) solvent alone was dispensed and then vaporized. The dendritic cells derived from bone marrow cells were applied to the plate onto which GSL had been immobilized, and the culture supernatant was recovered 24 hours later. The amount of IL-12 p70 and the amount of IL-10 in the culture supernatant was measured by ELISA using the BD Opt EIA kit. The results of the measurement of the amount of IL-12 p70 are shown in Table 3, and those of IL-10 are shown in Table 4.

**Table 3**

| Sample | IL-12p70 (pg/ml) |
|---|---|
| Control group | Lower than the detection limit |
| GSL-1 | 79.3 |
| GSL-2 | 103.4 |
| GSL-4 | 231.2 |
| GSL-6 | 296.6 |
| GSL-8 | 454.0 |
| GSL-10 | 547.0 |
| GSL-12 | 868.6 |

**Table 4**

| Sample | IL-10 (pg/ml) |
|---|---|
| Control group | 129.0 |
| GSL-4 | 147.4 |
| GSL-10 | 431.1 |
| GSL-12 | 583.2 |
| GSL-13 | 155.9 |

As is apparent from the tables, induction of IL-12 p70 and induction of IL-10 were observed. In particular, for induction of IL-12 p70, GSL-2, 4, 6, 8, 10, and 12 were found to be more effective, and GSL-6, 8, 10, and 12 were found to be remarkably effective. For induction of IL-10, GSL-10 and 12 were found to be remarkably effective.

### Example 5: Effects of NK cell activation

The C57BL/6 mice (7-week-old, female) were used. Each type of GSL was dissolved in the same manner as in Example 2. The samples were administered to mice through the caudal veins (dose: 100 µg). As the control, a cell suspension was prepared from the spleen extracted from the control mice to which the solvent alone had been administered 16 hours after the administration. The spleen cells were mixed with the ⁵¹Cr-labeled YAC-1 cells at a 50:1 ratio, and those cells were then cultured for 4 hours. The amount of ⁵¹Cr in the culture supernatant 4 hours later was measured using a γ-counter (Wallac). The results are shown in Table 5. This experiment was separately carried out three times, i.e., experiment 1, experiment 2, and experiment 3.

**Table 5**

| | Sample | NK cell activity (%) (mean ± S.D.) |
|---|---|---|
| Experiment 1 | Control | 26.1 ± 5.1 |
| | GSL-2 | 34.1 ± 6.2 |
| | GSL-3 | 33.2 ± 8.2 |
| | GSL-5 | 35.7 ± 4.8 |
| Experiment 2 | Control | 17.7 ± 2.0 |
| | GSL-1 | 30.1 ± 3.8 |
| | GSL-6 | 27.3 ± 4.5 |
| | GSL-7 | 33.5 ± 5.1 |
| Experiment 3 | Control | 8.3 ± 3.6 |
| | GSL-8 | 22.2 ± 1.7 |
| | GSL-9 | 14.8 ± 2.1 |
| | GSL-10 | 12.5 ± 3.0 |
| | GSL-12 | 11.2 ± 3.4 |
| | GSL-13 | 14.4 ± 3.1 |

As is apparent from Table 5, GSL-1, 5, 6 to 9, and 13 were found to be more effective, and GSL-1, 7, and 8 were found to be remarkably effective.

### Example 6: Antitumor effects

The C57BL/6 mice (7-week-old, female) were used. F16 melanoma F10(F16F10) tumor cells (the Institute for Genetic Medicine, Hokkaido University) were administered to mice through the caudal veins (2 x 10⁵ cells/mouse). Each type of GSL was dissolved in the same manner as in Example 2. The dose of GSL was 100 µg, and administration thereof was carried out intraperitoneally 1, 5, and 9 days after the administration of tumor cells. The mice were subjected to dissection 14 days after the administration of tumor cells, and the number of metastatic foci in the lung was counted. The results are shown in Table 6.

**Table 6**

| Sample (solvent) | Number of metastatic foci (mean ± S.D.) |
|---|---|
| Control | 111.2 ± 17.5 |
| GSL-1 (P) | 89.5 ± 15.9 |
| GSL-2 (P) | 40.0 ± 15.1 |
| GSL-6 (P) | 63.2 ± 21.8 |
| GSL-1 (P+S) | 69.7 ± 16.9 |
| GSL-2 (P+S) | 55.7 ± 19.8 |
| GSL-6 (P+S) | 68.7 ± 19.9 |
| GSL-7 (P+S) | 67.5 ± 14.7 |

As is apparent from Table 6, all the above GSLs effectively inhibited the lung metastases. The effects of GSL-2 were particularly remarkable.

### Example 7: Antiallergic effects

The mixed antigens of 100 µg of ovalbumin (OVA) and 1.6 mg of aluminum hydroxide gel were administered via subcutaneous vaccination to the BALB/c mice on days 0 and day 7. OVA (10 µg) was dissolved in physiological saline. The OVA (10 µg) dissolved in physiological saline were administered via nasal vaccination to the mice on days 14, 15, and 16. The mice were dissected 3 days after the nasal administration thereof (18 days after the initial administration). Each type of GSL was dissolved in a solution containing 0.5% pentanediol, 0.5% N-lauroylsarcosine, and 9.8% sucrose to a final concentration of 5 mg/ml and then diluted in physiological saline. The GSL solution diluted in physiological saline was administered intraperitoneally to mice on days 1, 5, 9, and 13. The total cell count and eosinophilic leukocyte count in the alveoli washing fluids were determined. The total cell count was determined by staining the cells with Turk solution (Wako Pure Chemical Industries, Ltd.). The eosinophilic leukocyte count was determined by staining the cells with Giemsa stain (Merck). The anti-OVA antibody titers (IgG, IgG1, and IgG2a) were assayed by adding the gradually diluted serum to a 96-well plate coated with OVA. Alkaline phosphatase-labeled anti-mouse IgG antibodies (Zymed), anti-mouse IgG1 antibodies (ICN), and anti-mouse-IgG2a antibodies (Zymed) were used as detection antibodies. The color was developed using paranitrophenyl phosphate as a substrate, the reaction was terminated with 3N NaOH, and the absorbance at 405 nm was measured using a microplate reader. The anti-OVA IgE antibody titer was assayed by adding the gradually diluted serum to a 96-well plate coated with the anti-mouse IgE antibodies (Nippon Becton Dickinson Co., Ltd.). Subsequently, biotin-labeled OVA was added to each well, and the color was developed using peroxidase-labeled streptavidin and SureBlue (Funakoshi) as a substrate. After the reaction was terminated with 2N H₂PO₄, the absorbance at 405 nm was measured using a microplate reader.

The control group (the BALB/c mice) was subjected to the experiment at the same time without GSL administration.

The results of determining leukocyte count are shown in Table 7, and those of eosinophilic leukocyte count are shown in Table 8.

**Table 7**

| | Leukocyte count 1×10⁵ cells/ml (mean ± S.D.) |
|---|---|
| Normal mice | 32.7 ± 9.9 |
| Control | 147.0 ± 50.1 |
| GSL-1 | 88.5 ± 34.6 |

**Table 8**

| | Eosinophilic leukocyte count 1×10⁵ cells/ml (mean ± S.D.) |
|---|---|
| Normal mice | 0 |
| Control | 27.7 ± 24.1 |
| GSL-1 | 4.9 ± 4.2 |

As is apparent from Table 7 and Table 8, the leukocyte count and the eosinophilic leukocyte count in the alveoli washing fluids were significantly decreased.

### Example 8: Effects of enhancing resistance to infection

The C57BL/6 mice (7-week-old, female) were used. Each type of GSL was dissolved in the same manner as with the case of "P" in Example 2. The solution was administered intraperitoneally to mice 1 hour before infection with Salmonella typhimurium SL7207 aroA (Stanford University School of Medicine, U.S.A.). The mice were dissected 3 days after the infection, and the intraperitoneal viable cell count was determined. The results are shown in Table 9.

**Table 9**

| Sample | Intraperitoneal viable cell count ×10⁶ cfu (mean ± S.D.) |
|---|---|
| Control | 20.9 ± 3.5 |
| GSL-1 | 12.1 ± 8.2 |
| GSL-2 | 6.7 ± 3.6 |

In Table 9, "cfu" is an abbreviation for "colony forming unit." As is apparent from Table 9, administration of GSL enhanced the resistance to infection.

### Example 9: Antiviral effects

The C57BL/6 mice (7-week-old, female) were used. Each type of GSL was dissolved in the same manner as in Example 2. The solution was administered intravenously to mice. Mouse cytomegalovirus (strain Smith, 1 x 10⁴ pfu; "pfu" is an abbreviation for "plaque forming unit") was inoculated intraperitoneally to mice 1 hour after the administration. The mice were dissected 3 days after the infection, and the viral titers in the liver and in the spleen were determined. The results are shown in Table 10. NK cell activity and serum IFN-γ in the spleen were measured 3 days after the infection. The results are shown in Table 11 (NK cell activity) and in Table 12 (IFN-γ).

**Table 10**

| Sample | Virus in spleen ×10³pfu (mean ± S.D.) | Virus in liver ×10³pfu (mean ± S.D.) |
|---|---|---|
| Control | 2.4 ± 0.5 | 63.0 ± 19.5 |
| GSL-1 | 0.2 ± 0.1 | 7.5 ± 4.1 |
| GSL-2 | 0.2 ± 0.1 | 3.3 ± 1.9 |
| GSL-6 | 0.2 ± 0.1 | 2.7 ± 1.2 |
| GSL-7 | 0.2 ± 0.1 | 4.8 ± 1.9 |

**Table 11**

| Sample | NK cell activity (mean ± S.D.) |
|---|---|
| Control | 41.4 ± 10.7 |
| GSL-1 | 72.5 ± 9.7 |
| GSL-2 | 54.0 ± 14.0 |
| GSL-6 | 75.2 ± 4.8 |
| GSL-7 | 72.5 ± 7.4 |

**Table 12**

| Sample | Serum IFN-γ (ng/ml) (mean ± S.D.) |
|---|---|
| Control | 2.0 ± 0.5 |
| GSL-1 | 3.0 ± 0.7 |
| GSL-2 | 4.5 ± 1.1 |
| GSL-6 | 2.6 ± 0.7 |
| GSL-7 | 2.5 ± 0.3 |

As shown in Table 10, pre-administration of GSL resulted in the enhancement of resistance to infection. As shown in Table 11, NK cell activity was significantly enhanced, and the IFN-γ level was enhanced, compared with the control group. The effects of GSL-6 were found to be particularly significant.

### Example 10: Acceleration of IL-6 production and acceleration of NO production

Thioglycolate acid (4%, 3 ml) was administered intraperitoneally to the normal mice that were employed in example 1, and the peritoneal exudate cells were sampled therefrom 4 days later and then subjected to the experiment. The macrophage cell line RAW 264 (RIKEN) was also subjected to the experiment. Each type of GSL was dissolved in an ethanol:dodecane (98:2) solvent at 0.5 mg/ml, 20 µl each thereof was dispensed into each well of a 96-well plate, the solvent was vaporized, and the GSL was immobilized onto the plate. The macrophage was added to the plate onto which GSL had been immobilized, and the culture supernatant was recovered 24 hours thereafter. The amount of IL-6 in the supernatant was measured via ELISA and the amount of nitrogen monoxide (NO) was measured using the Griess reagent. The results of measuring IL-6 are shown in Table 13, and those of measuring nitrogen monoxide are shown in Table 14.

**Table 13**

| Sample | IL-6 (pg/ml) (mean ± S.D.) |
|---|---|
| Control | 3491.0 ± 492.1 |
| GSL-1 | 3921.7 ± 665.1 |
| GSL-4 | 3453.0 ± 252.5 |
| GSL-5 | 4742.0 ± 525.2 |
| GSL-6 | 5389.7 ± 1003.7 |
| GSL-7 | 6948.0 ± 457.1 |
| GSL-8 | 6264.0 ± 1075.0 |
| GSL-9 | 6711.3 ± 485.1 |
| GSL-10 | 7474.0 ± 920.3 |
| GSL-11 | 10046.0 ± 340.0 |
| GSL-12 | 7303.3 ± 640.2 |
| GSL-13 | 7619.3 ± 774.3 |

**Table 14**

| Sample | NO (µM) (mean ± S.D.) |
|---|---|
| Control group | 0.7 ± 0.2 |
| GSL-2 | 6.8 ± 0.7 |
| GSL-3 | 3.5 ± 0.2 |
| GSL-4 | 3.8 ± 0.5 |
| GSL-5 | 5.2 ± 0.5 |
| GSL-6 | 4.4 ± 0.8 |
| GSL-7 | 3.9 ± 0.3 |
| GSL-8 | 4.2 ± 1.1 |
| GSL-9 | 9.0 ± 0.5 |
| GSL-10 | 8.4 ± 0.8 |
| GSL-11 | 11.3 ± 1.0 |
| GSL-12 | 7.7 ± 0.5 |
| GSL-13 | 7.1 ± 0.1 |

As is apparent from Table 13, effects of IL-6 induction were observed. GSL-5 to 13 were found to be more effective, GSL-6 to 13 were found to be further effective, and GSL-7 to 13 were found to be remarkably effective.

As is apparent from Table 14, effects of accelerating NO production were observed. GSL-2, 5, and 9 to 13 were found to be more effective, and GSL-11 was further effective.

### Example 11

The toxicities of the GSL compositions according to the present invention were examined. The C57BL/6 mice (7-week-old, female) were used. Galactosamine (20 mg) was administered intraperitoneally to mice, and immediately thereafter, lipopolysaccharides (hereafter the same may be abbreviated as "LPS," derived from Salmonella abortus equi, the Max-Planck Institute for Immunobiology, Freiburg, Germany) (solvent: physiological saline) or GSL shown in Table 15 (solvent: "P+S") were administered to mice via the caudal veins in amounts shown in Table 15. Whether or not the mice survived was examined 24 hours after the administration. The results are shown in Table 15.

**Table 15**

| LPS or GSL | Amount administered | Number of mice died/Number of mice tested |
|---|---|---|
| GSL-1 | 100 µg | 0/3 |
| GSL-2 | 100 µg | 0/3 |
| GSL-3 | 100 µg | 0/3 |
| GSL-4 | 100 µg | 0/3 |
| GSL-5 | 100 µg | 0/3 |
| GSL-6 | 100 µg | 0/3 |
| GSL-7 | 100 µg | 0/3 |
| GSL-8 | 100 µg | 0/3 |
| GSL-9 | 100 µg | 0/3 |
| GSL-10 | 100 µg | 0/3 |
| GSL-11 | 100 µg | 0/3 |
| GSL-12 | 100 µg | 0/3 |
| GSL-13 | 100 µg | 0/3 |
| LPS | 100 ng | 3/3 |
| LPS | 10 ng | 3/3 |

When LPS was administered, all mice died even with the administration of 10 ng thereof. On the contrary, all mice survived with the administration of GSL in amounts of as much as 100 µg. Thus, the toxicities of the compositions according to the present invention were found to be very low.

### Example 10

Whether or not endotoxin tolerance was induced was examined. The C57BL/6 mice (7-week-old, female) were used. LPS (solvent: physiological saline) or GSL was administered to mice via the caudal veins in amounts shown in Table 16. Galactosamine (20 mg) and LPS (100 ng, the solvent: physiological saline) were administered intraperitoneally to mice 24 hours after the administration, and whether or not the mice survived was examined. The results are shown in Table 16.

**Table 16**

| LPS or GSL | Amount administered | Number of mice died/Number of mice tested |
|---|---|---|
| GSL-1 | 100 µg | 3/3 |
| GSL-2 | 100 µg | 3/3 |
| GSL-6 | 100 µg | 3/3 |
| GSL-7 | 100 µg | 3/3 |
| LPS | 100 ng | 0/3 |

As is apparent from Table 16, endotoxin tolerance was not induced with the administration of 100 µg of GSL-1, GSL-2, GSL-6, or GSL-7, and all mice died. On the contrary, endotoxin tolerance was induced with the administration of 100 ng of LPS.

It was thus found that the compositions of the present invention are more effective for NKT cell activation. Also, a composition for accelerating IL-4 production, a composition for accelerating IFN-γ production, a composition for dendritic cell activation, a composition for accelerating IL-12 production, a composition for accelerating IL-10 production, a composition for NK cell activation, an antitumor composition, an antiallergic composition, a composition for enhancing resistance to infection, an antiviral composition, a composition for accelerating IL-6 production, and a composition for accelerating NO production were obtained. The compositions of the present invention are particularly useful since they have such functions at once.

Further, the compositions of the present invention are useful since they have few side effects, i.e. the toxicities thereof are low and endotoxin tolerance is not induced.

## Claims

1. A composition for NKT cell activation comprising a glycosphingolipid having a structure represented by the following formula (1): 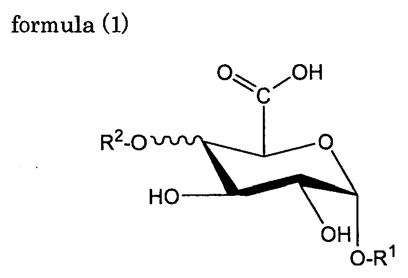 wherein R¹ represents the following formula (1-1): 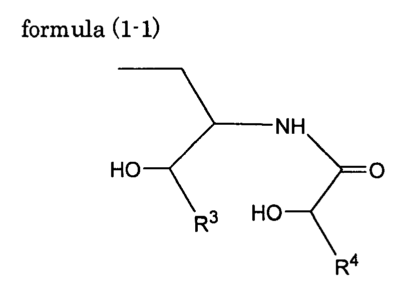 wherein R³ represents alkyl or alkenyl and R⁴ represents alkyl; and
R² represents hydrogen, or α-galactose, α-glucose, α-mannose, α-glucosamine, β-glucosamine or a combination thereof.

2. The composition for NKT cell activation according to Claim 1, wherein the formula (1) is represented by the following formula (3): 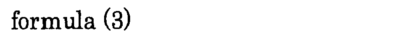 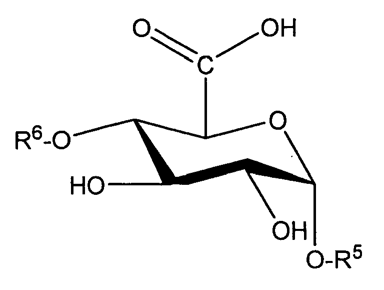 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

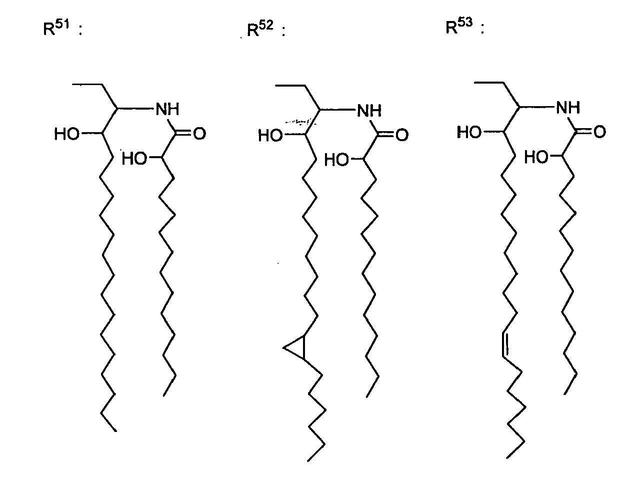

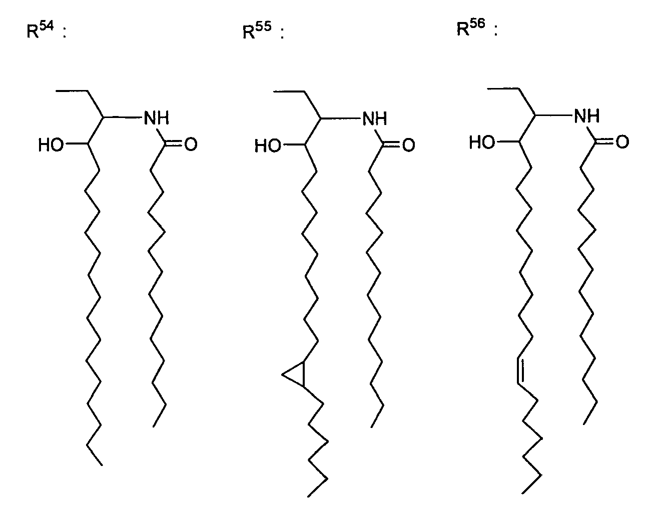

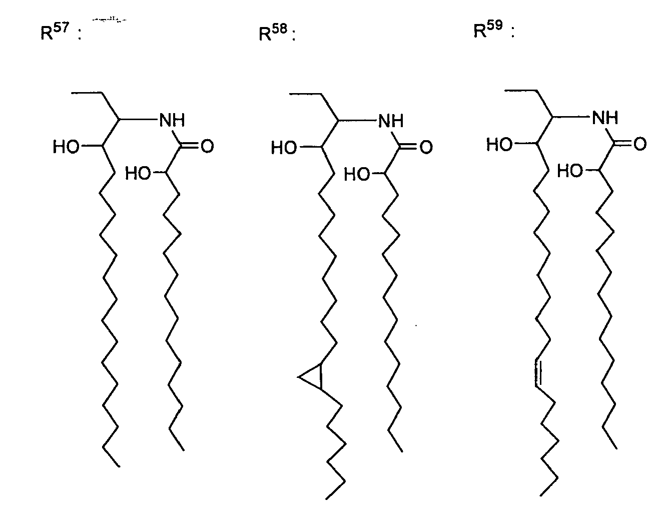

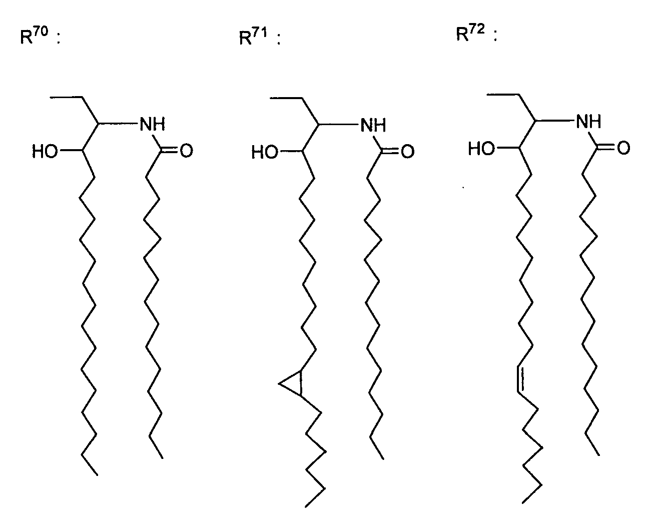

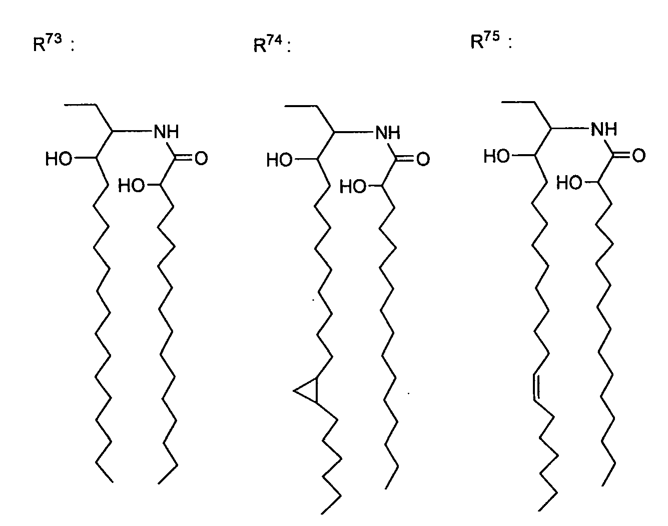

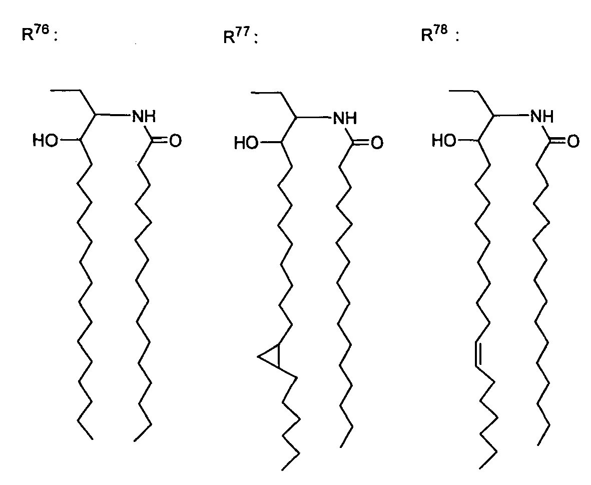

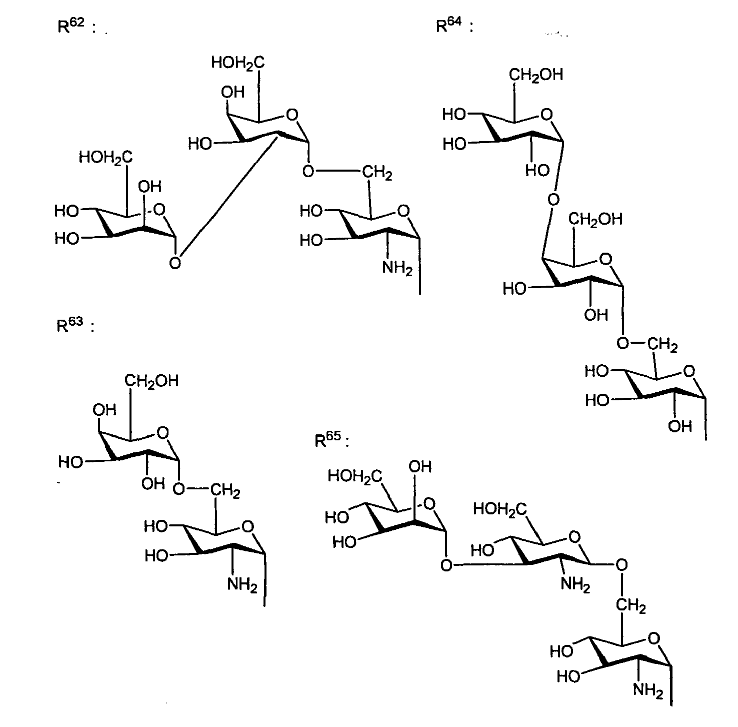

3. A composition for accelerating IL-4 production comprising a glycosphingolipid having a structure represented by the following formula (1): 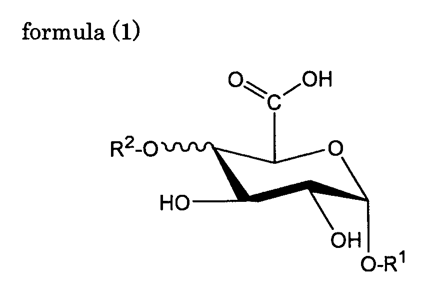 wherein R¹ represents the following formula (1-1): 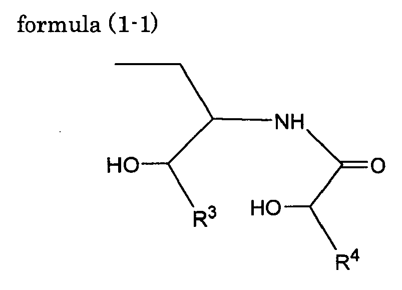 wherein R³ represents alkyl or alkenyl and R⁴ represents alkyl; and
R² represents hydrogen, or α-galactose, α-glucose, α-mannose, α-glucosamine, β-glucosamine or a combination thereof.

4. The composition for accelerating IL-4 production according to Claim 3, wherein the formula (1) is represented by the following formula (3): 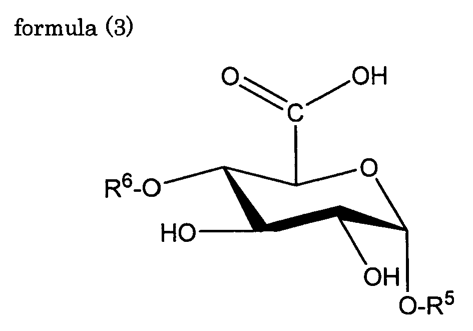 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

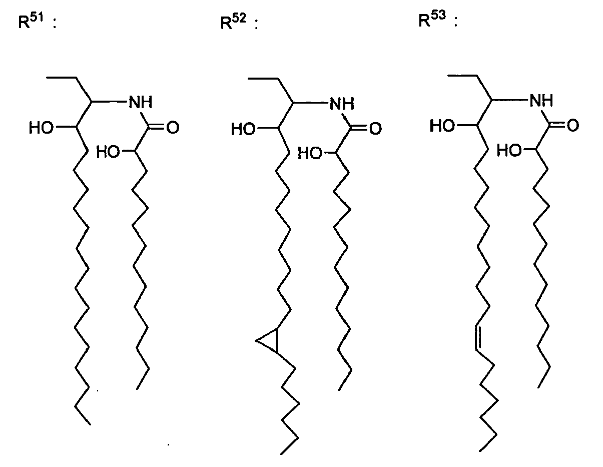

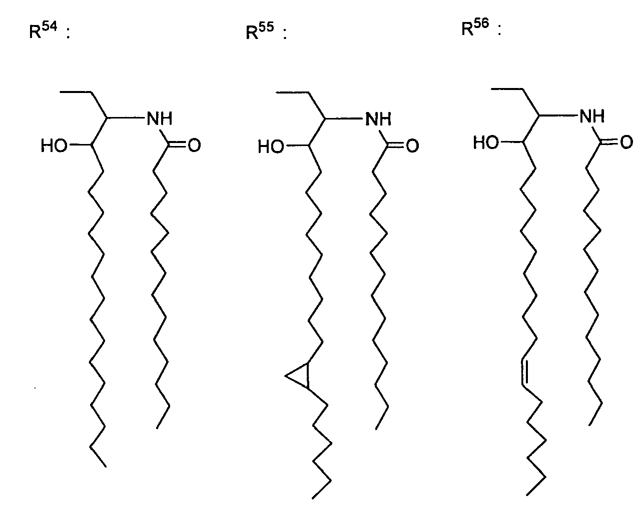

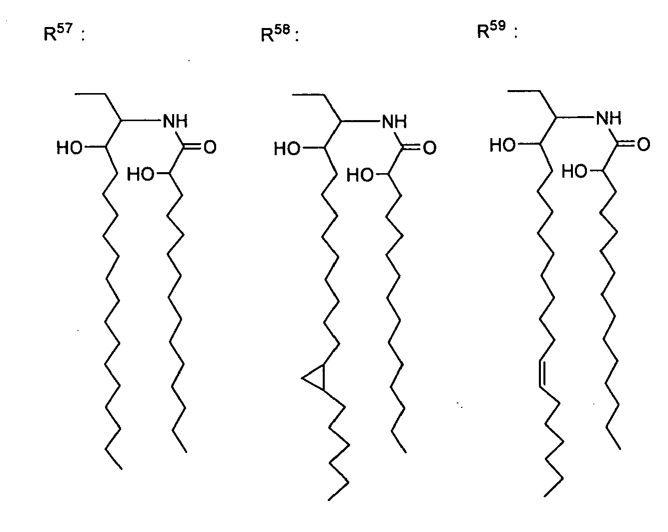

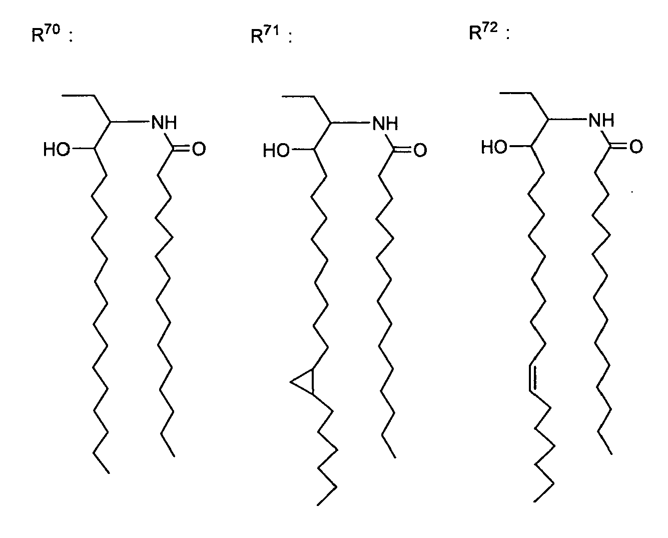

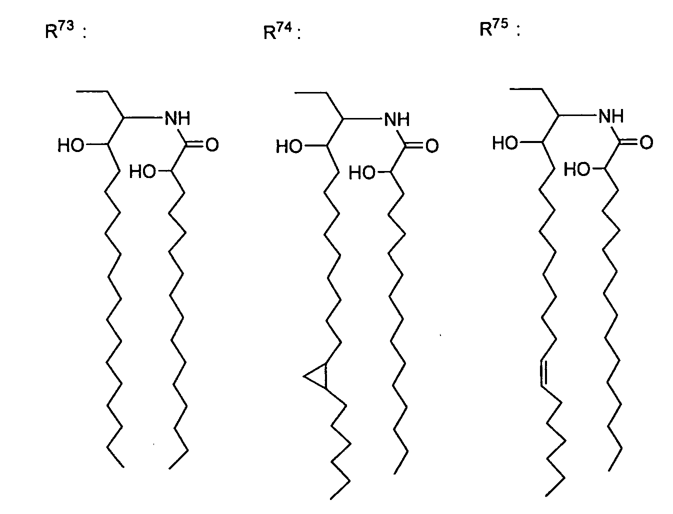

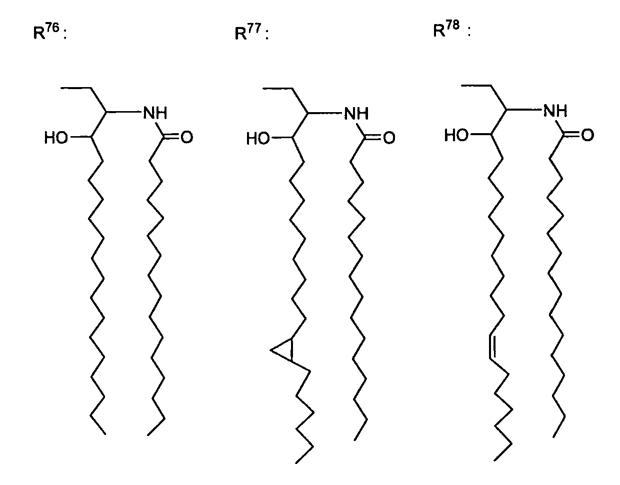

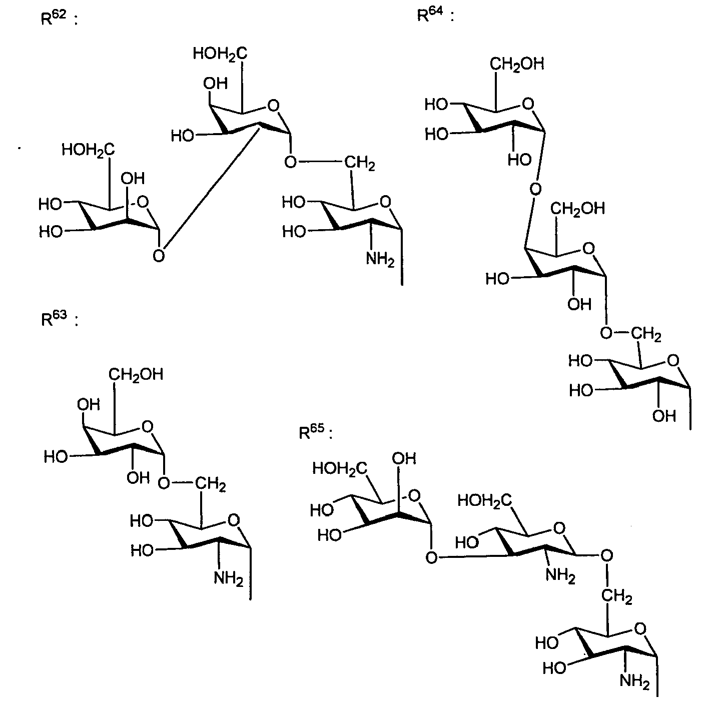

5. A composition for accelerating IFN-γ production comprising a glycosphingolipid having a structure represented by the following formula (1): 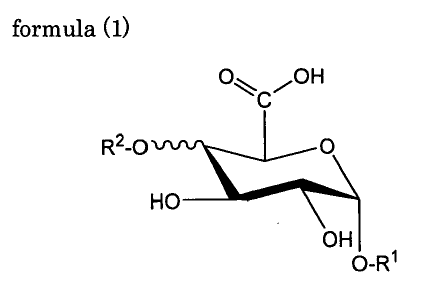 wherein R¹ represents the following formula (1-1): 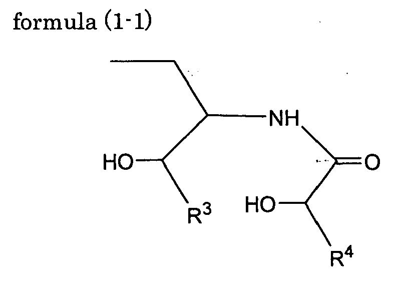 wherein R³ represents alkyl or alkenyl and R⁴ represents alkyl; and
R² represents hydrogen, or α-galactose, α-glucose, α-mannose, α-glucosamine, β-glucosamine or a combination thereof.

6. The composition for accelerating IFN-γ production according to Claim 5, wherein the formula (1) is represented by the following formula (3): 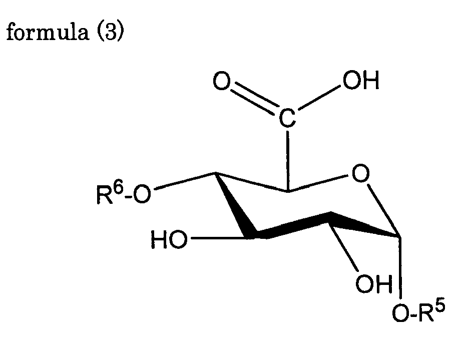 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

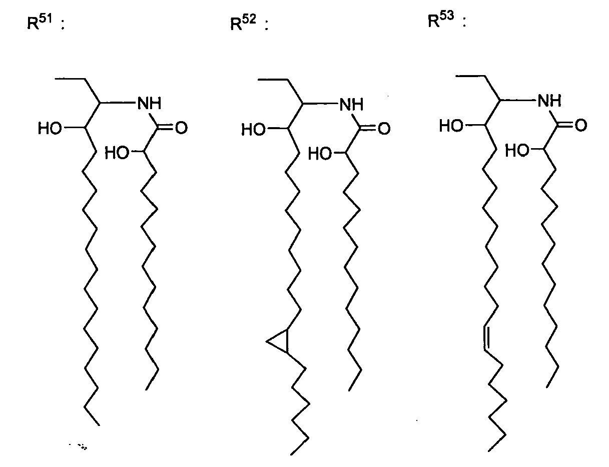

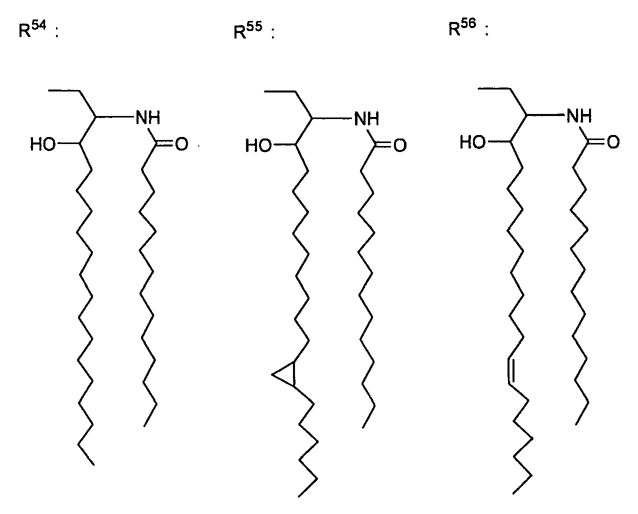

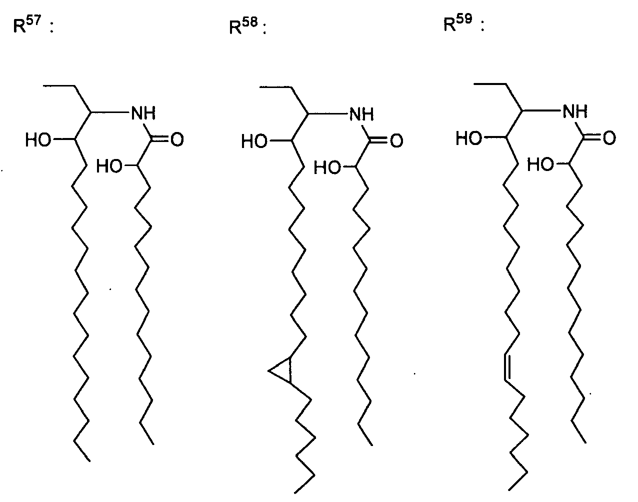

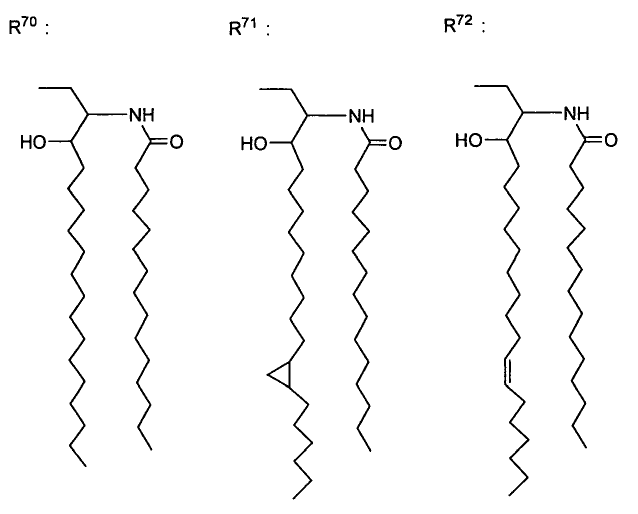

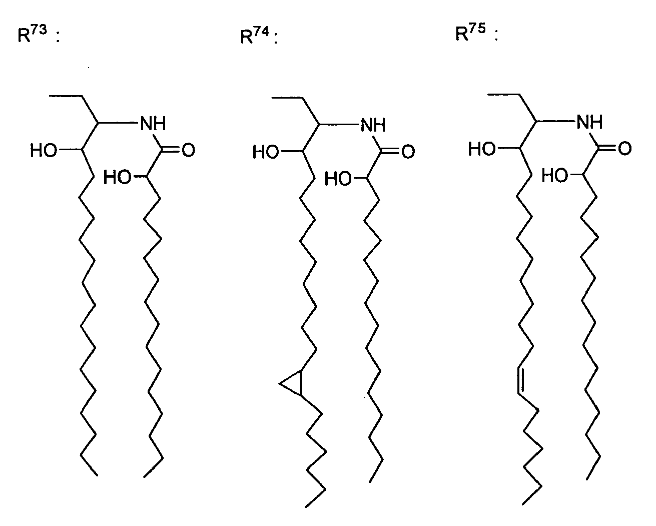

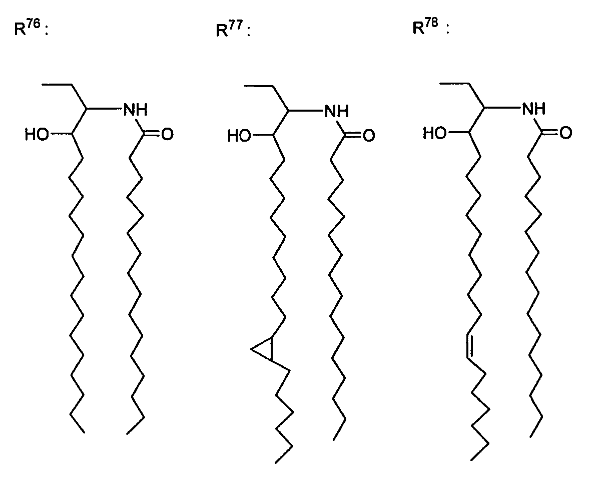

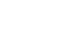

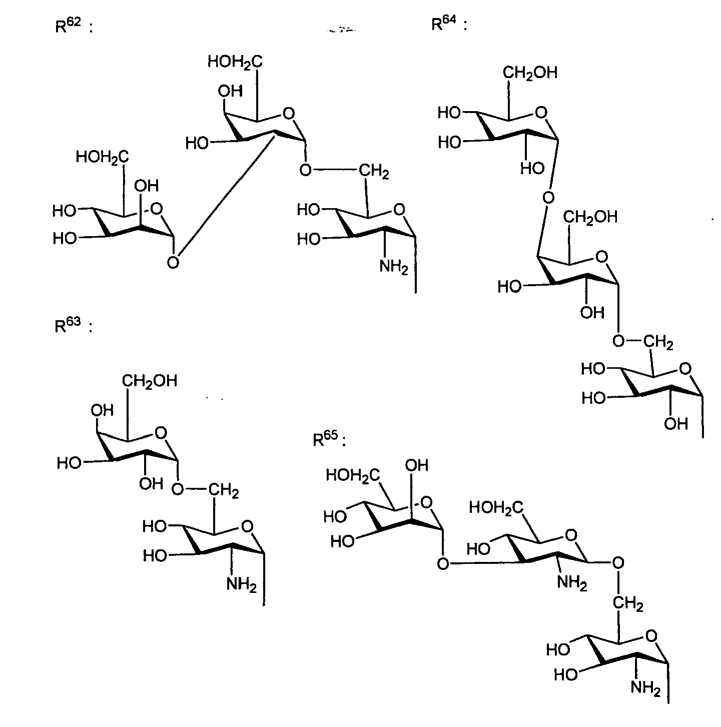

7. A composition for dendritic cell activation comprising a glycosphingolipid having a structure represented by the following formula (1): 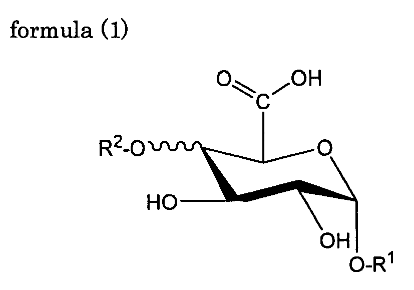 wherein R¹ represents the following formula (1-1): 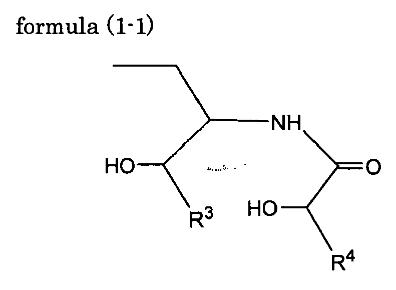 wherein R³ represents alkyl or alkenyl and R⁴ represents alkyl; and
R² represents hydrogen, or α-galactose, α-glucose, α-mannose, α-glucosamine, β-glucosamine or a combination thereof.

8. The composition for dendritic cell activation according to Claim 7, wherein the formula (1) is represented by the following formula (3): 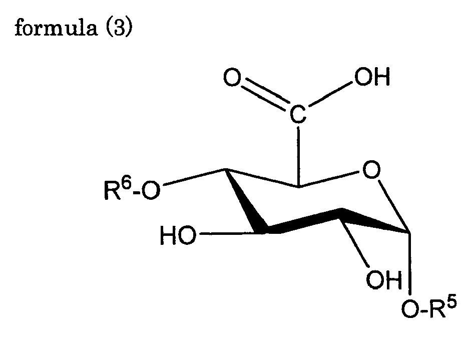 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

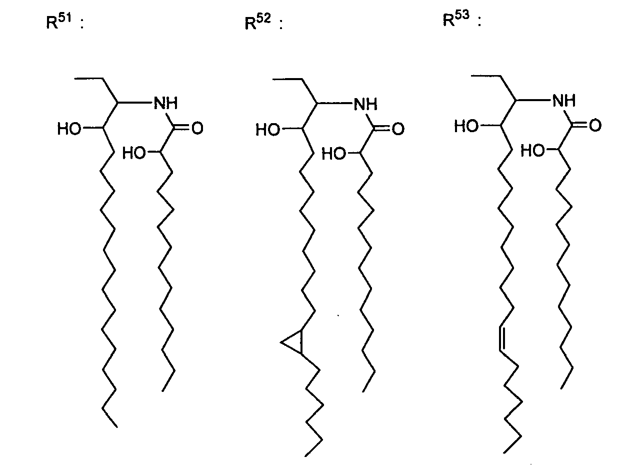

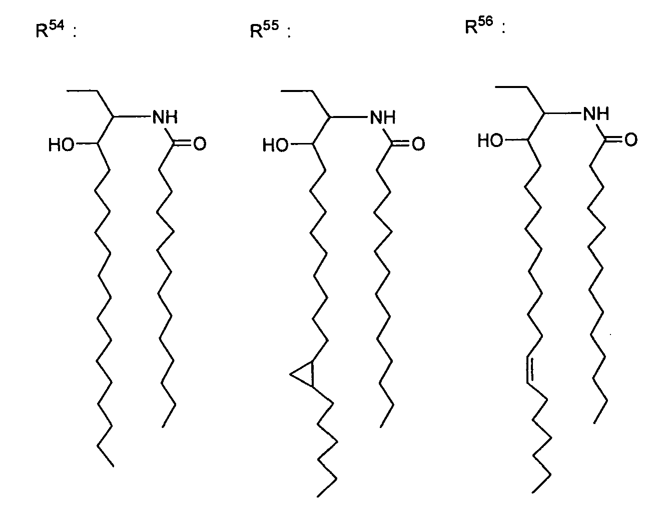

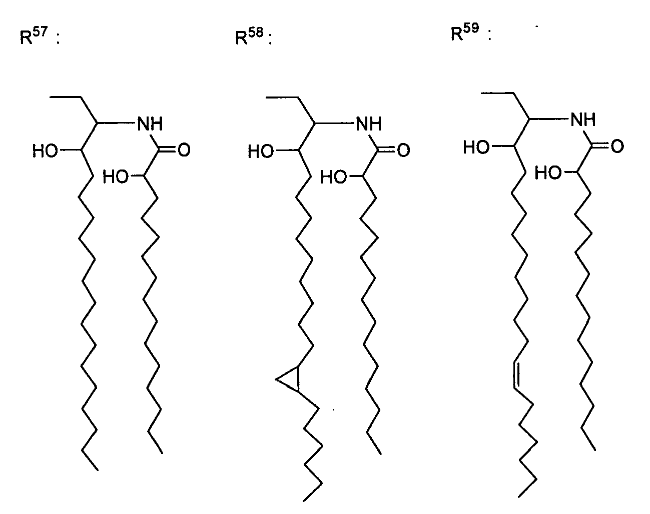

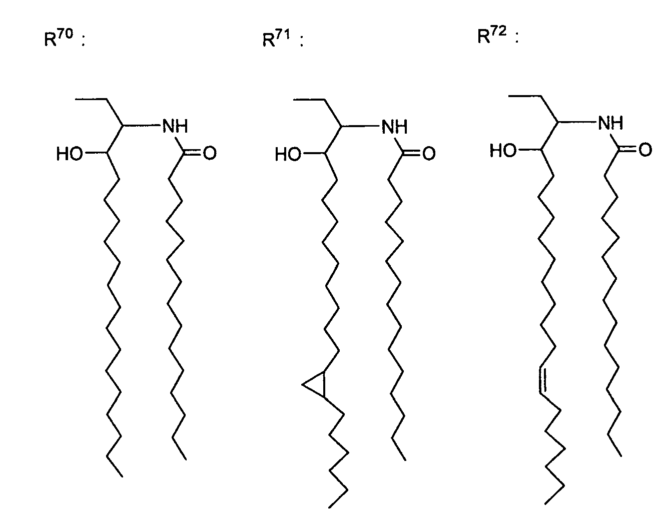

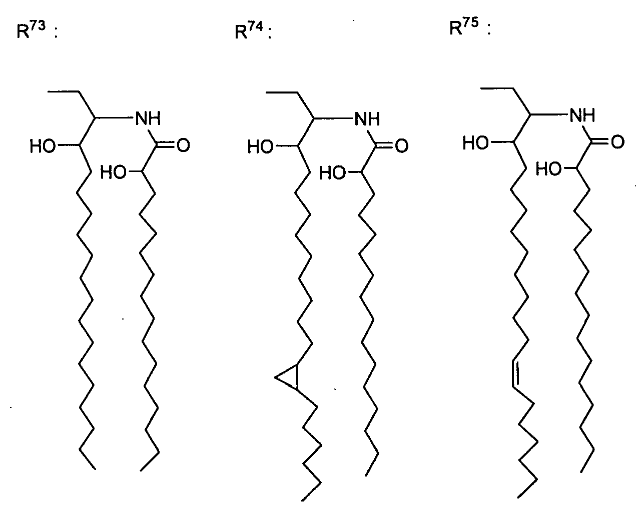

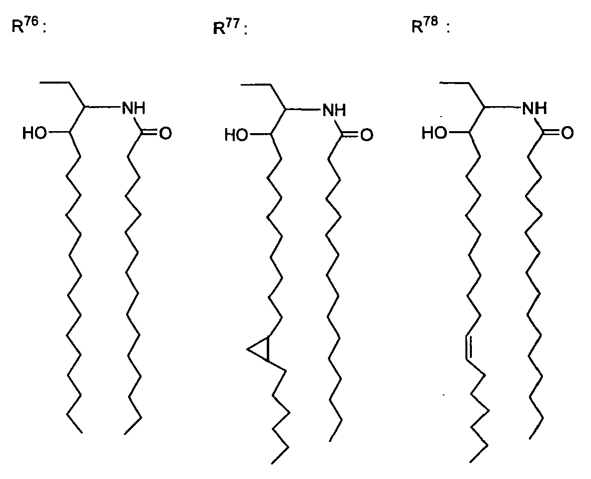

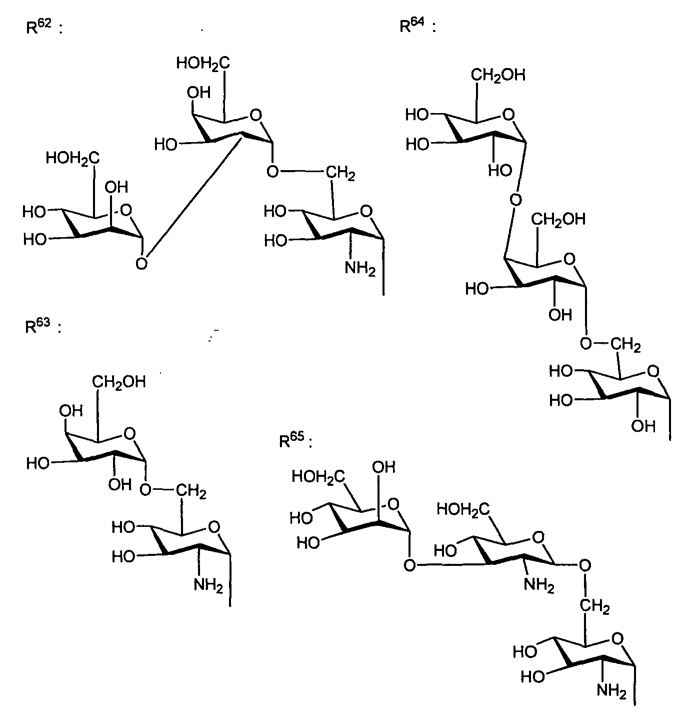

9. A composition for accelerating IL-12 production comprising a glycosphingolipid having a structure represented by the following formula (1): 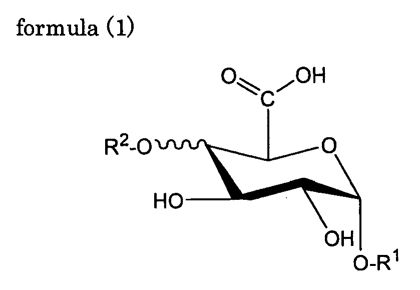 wherein R¹ represents the following formula (1-1): 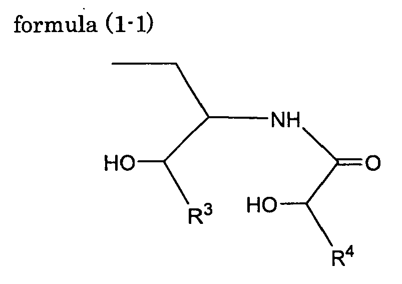 wherein R³ represents alkyl or alkenyl and R⁴ represents alkyl; and
R² represents hydrogen, or α-galactose, α-glucose, α-mannose, α-glucosamine, β-glucosamine or a combination thereof.

10. The composition for accelerating IL-12 production according to Claim 9, wherein the formula (1) is represented by the following formula (3): 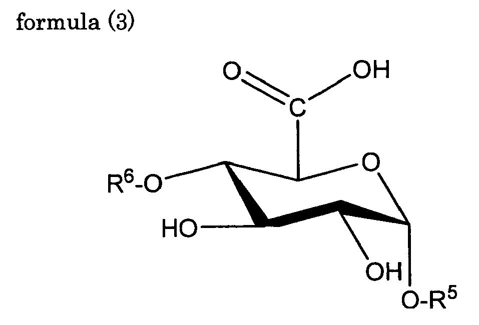 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

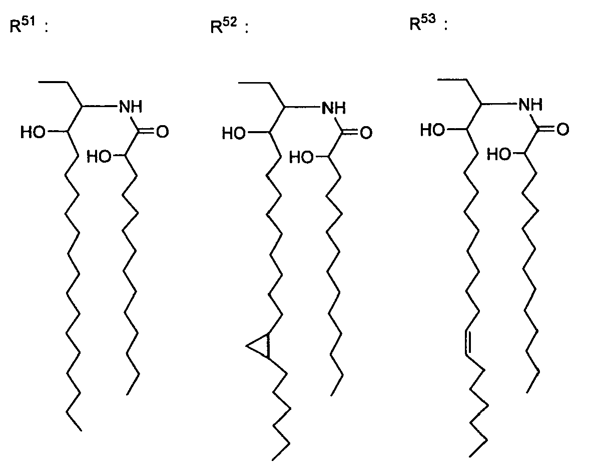

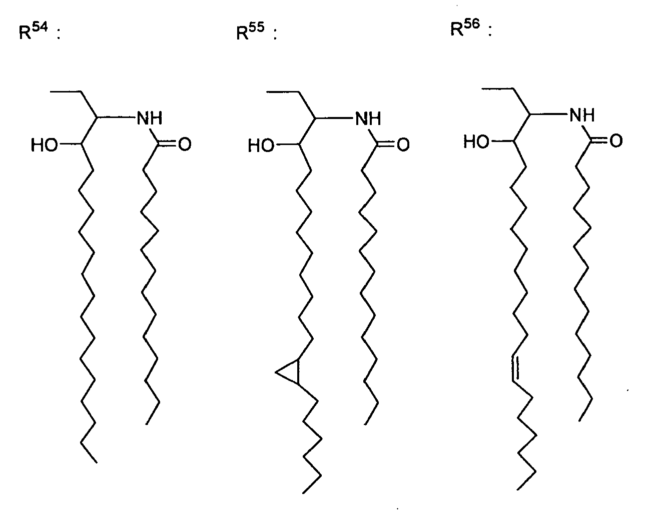

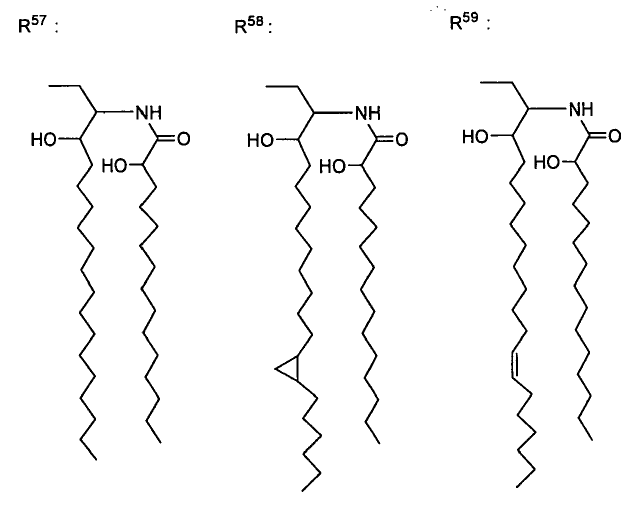

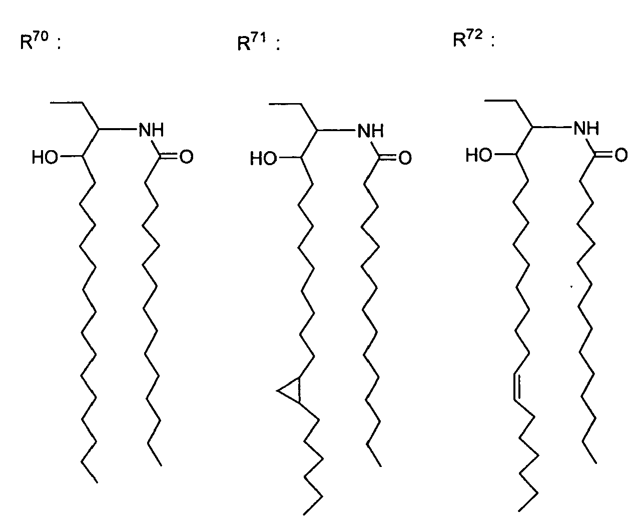

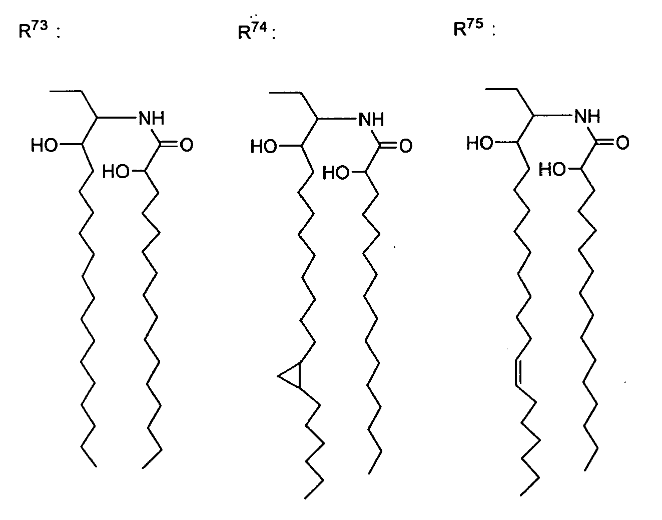

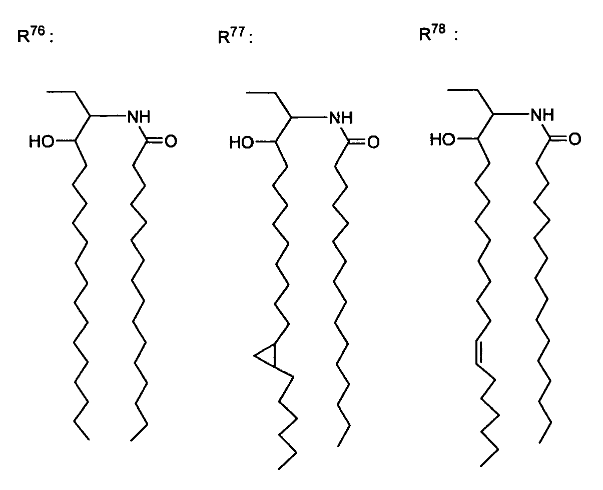

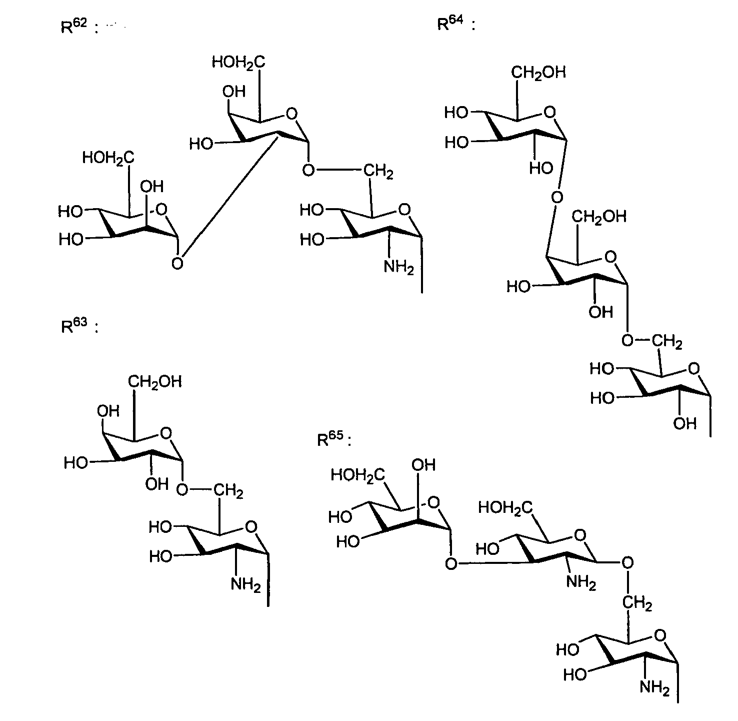

11. A composition for accelerating IL-10 production comprising a glycosphingolipid having a structure represented by the following formula (1): 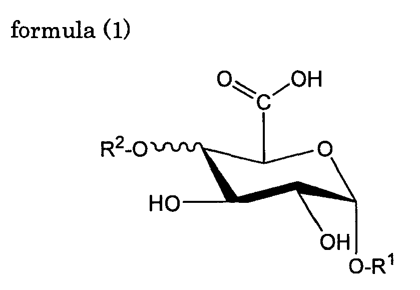 wherein R¹ represents the following formula (1-1): 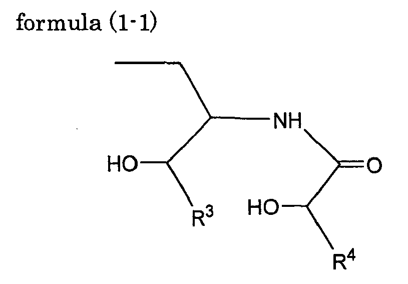 wherein R³ represents alkyl or alkenyl and R⁴ represents alkyl; and
R² represents hydrogen, or α-galactose, α-glucose, α-mannose, α-glucosamine, β-glucosamine or a combination thereof.

12. The composition for accelerating IL-10 production according to Claim 11, wherein the formula (1) is represented by the following formula (3): 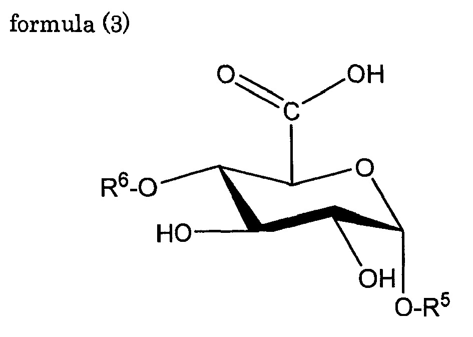 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

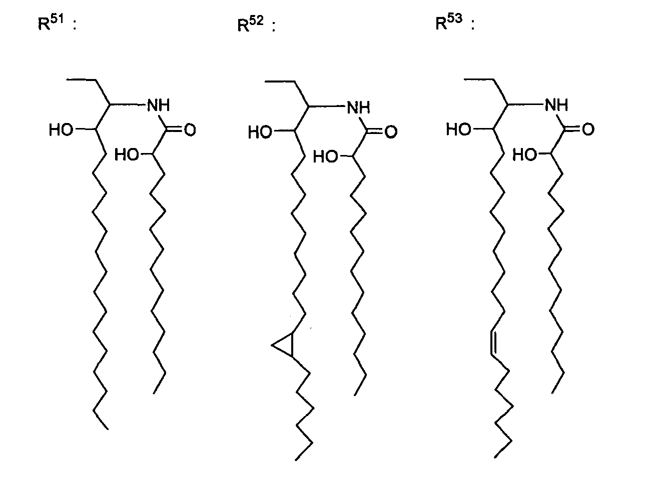

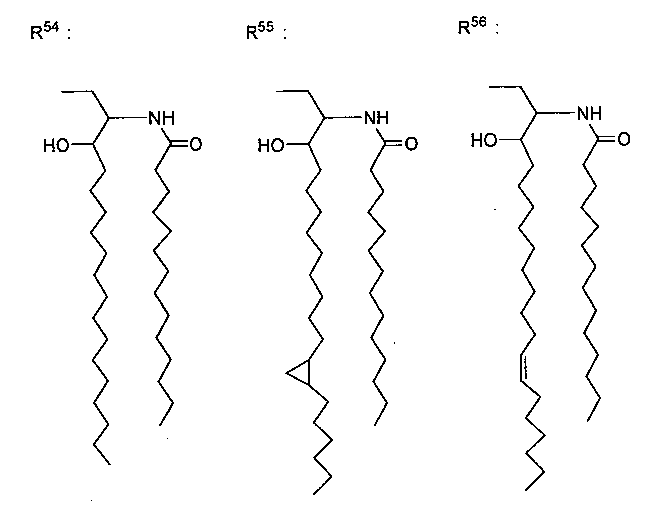

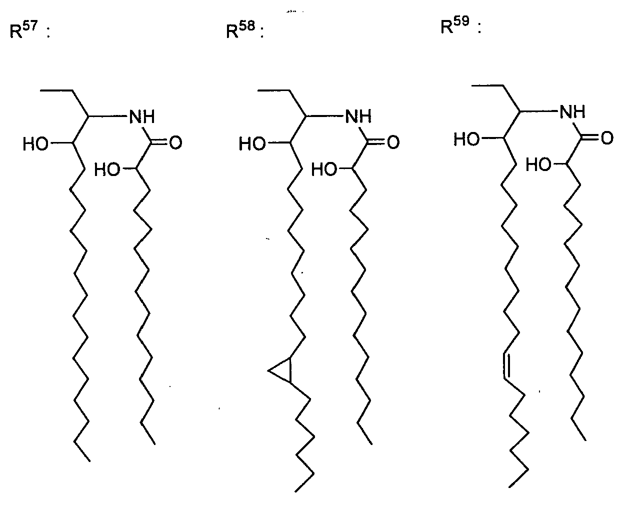

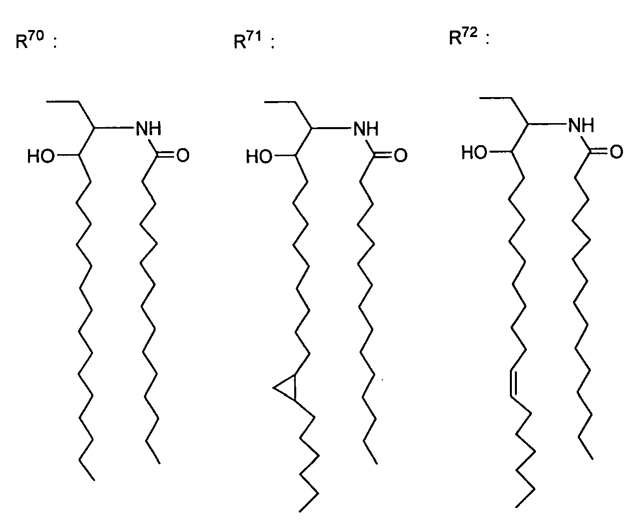

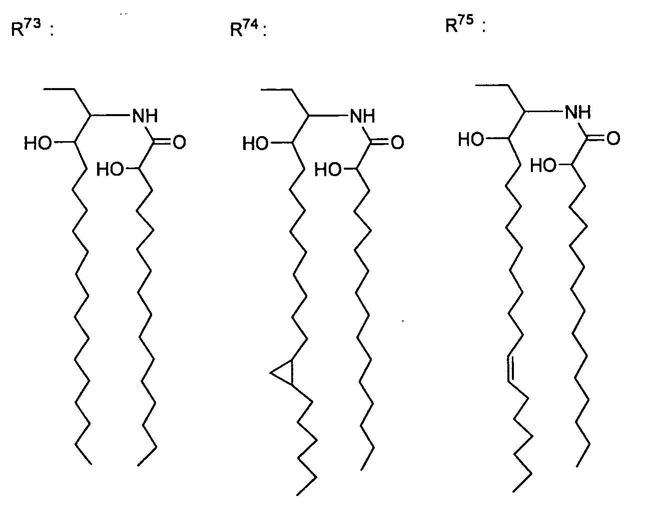

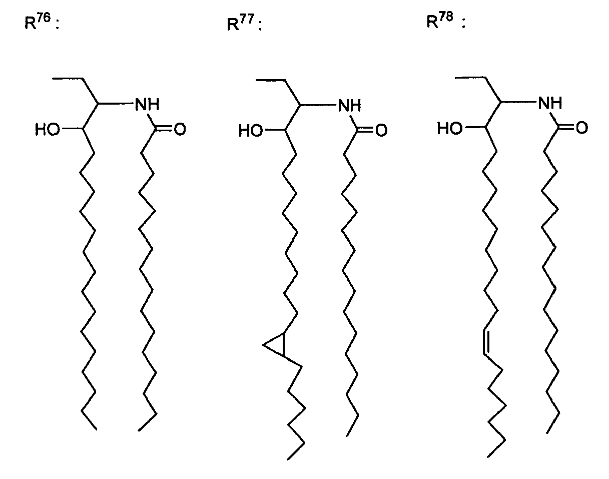

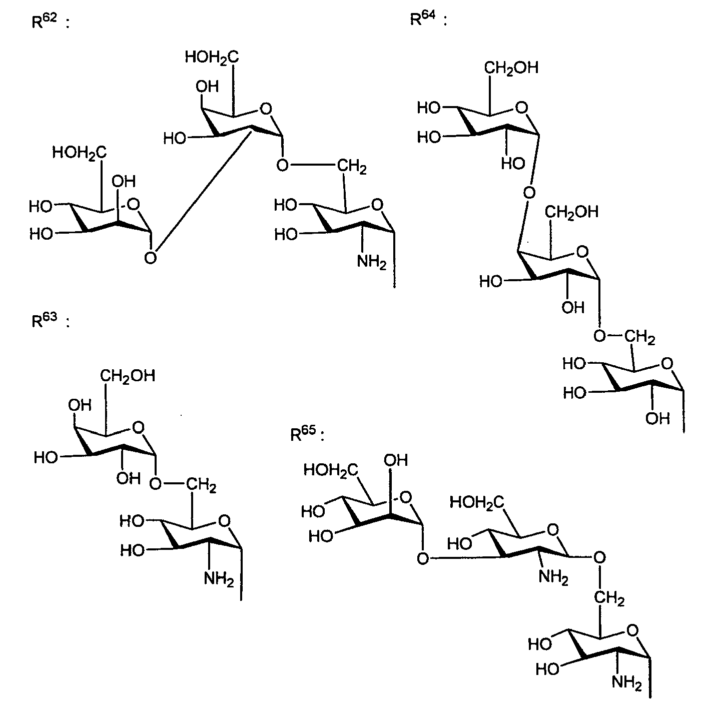

13. A composition for NK cell activation comprising a glycosphingolipid having a structure represented by the following formula (1): 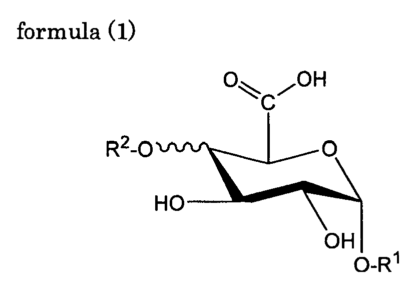 wherein R¹ represents the following formula (1-1): 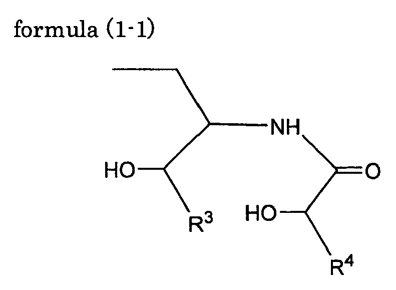 wherein R³ represents alkyl or alkenyl and R⁴ represents alkyl; and
R² represents hydrogen, or α-galactose, α-glucose, α-mannose, α-glucosamine, β-glucosamine or a combination thereof.

14. The composition for NK cell activation according to Claim 13, wherein the formula (1) is represented by the following formula (3): 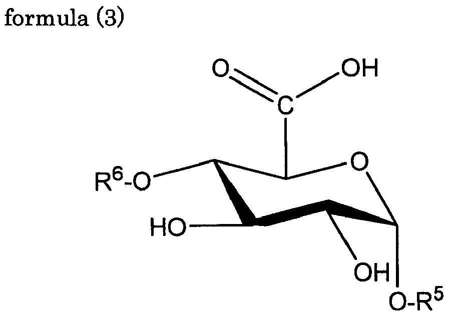 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

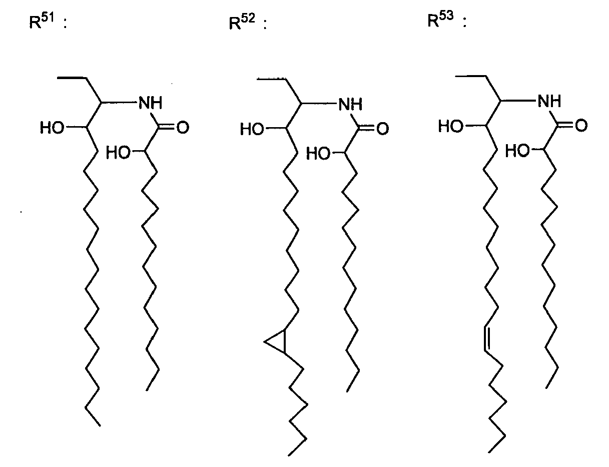

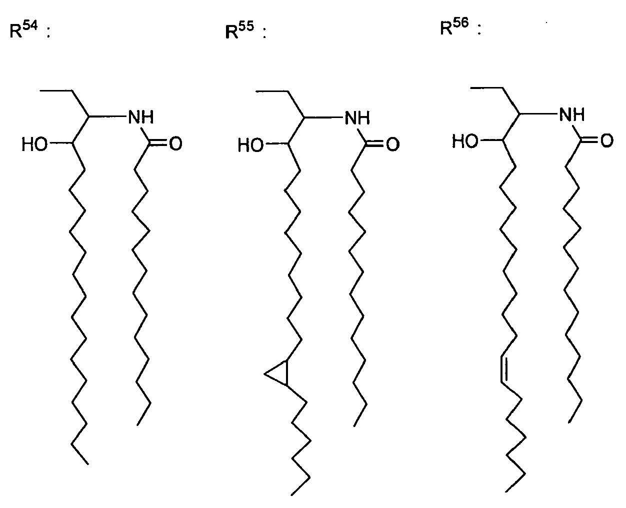

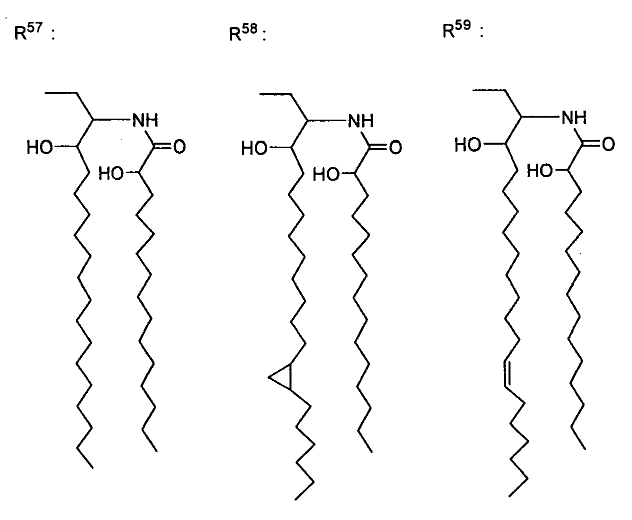

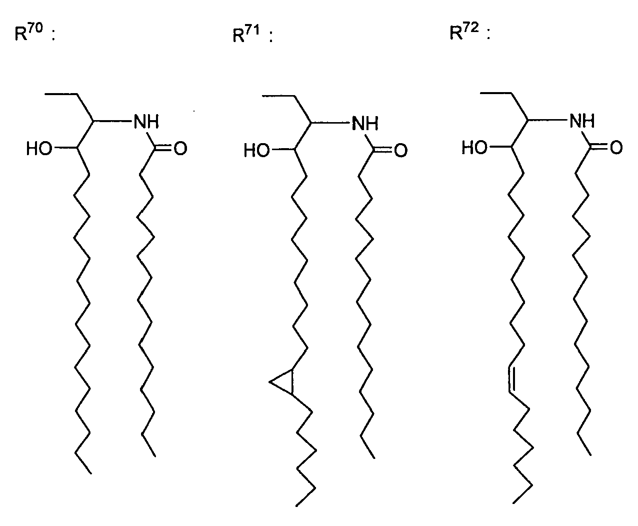

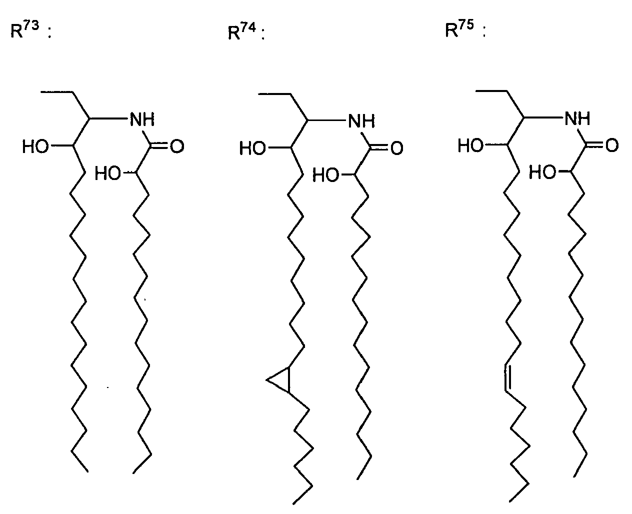

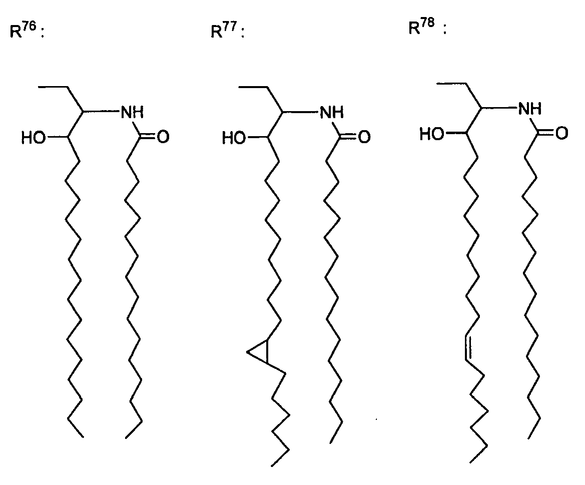

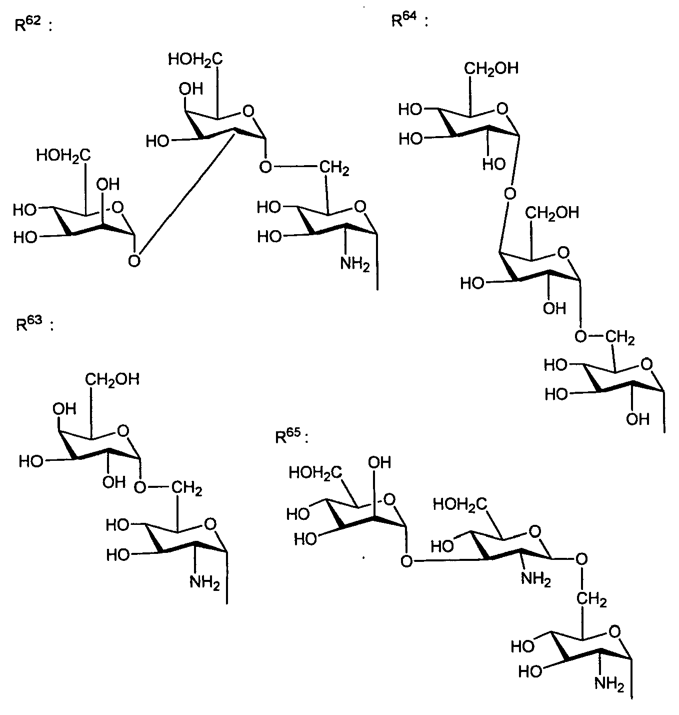

15. An antitumor composition comprising a glycosphingolipid having a structure represented by the following formula (1): 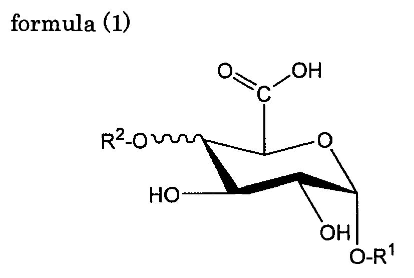 wherein R¹ represents the following formula (1-1): 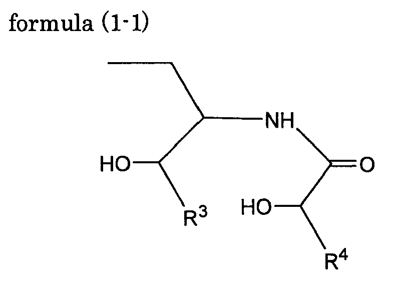 wherein R³ represents alkyl or alkenyl and R⁴ represents alkyl; and
R² represents hydrogen, or α-galactose, α-glucose, α-mannose, α-glucosamine, β-glucosamine or a combination thereof.

16. The antitumor composition according to Claim 15, wherein the formula (1) is represented by the following formula (3): 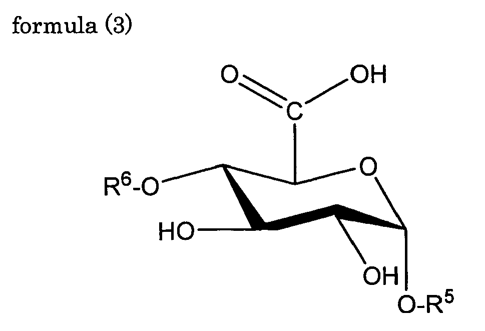 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

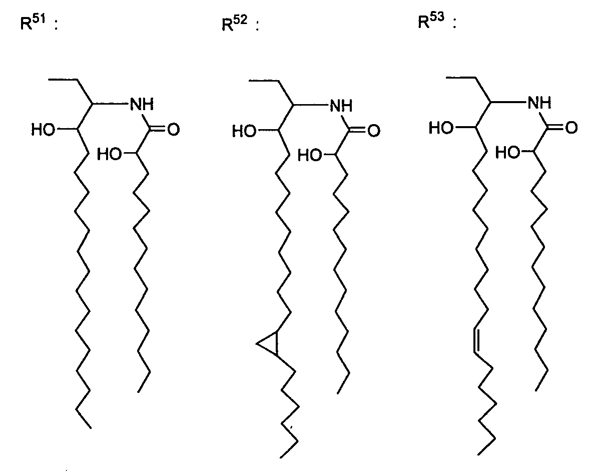

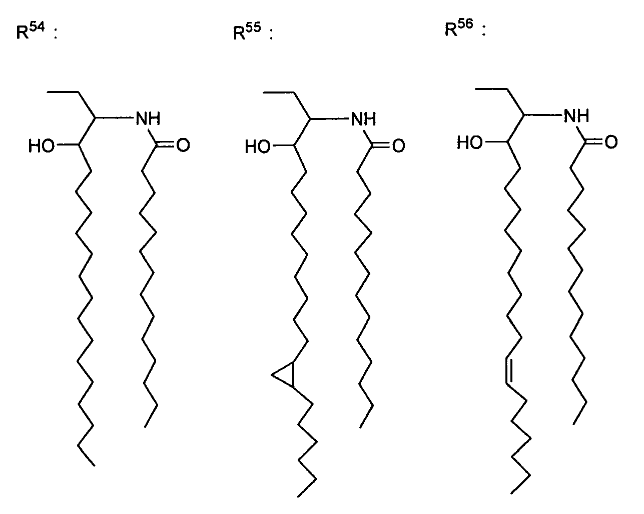

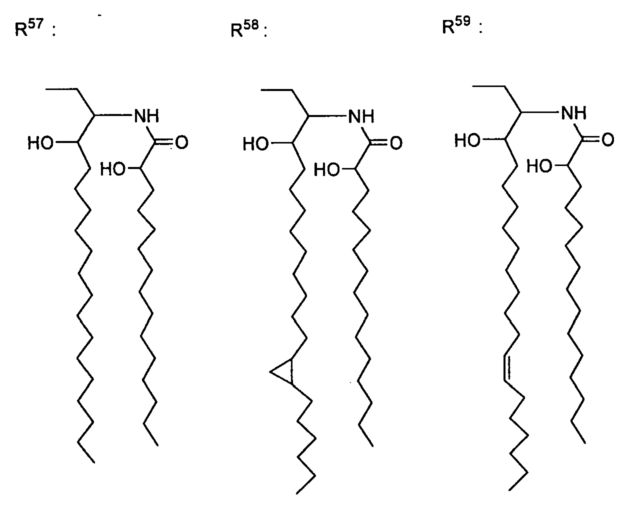

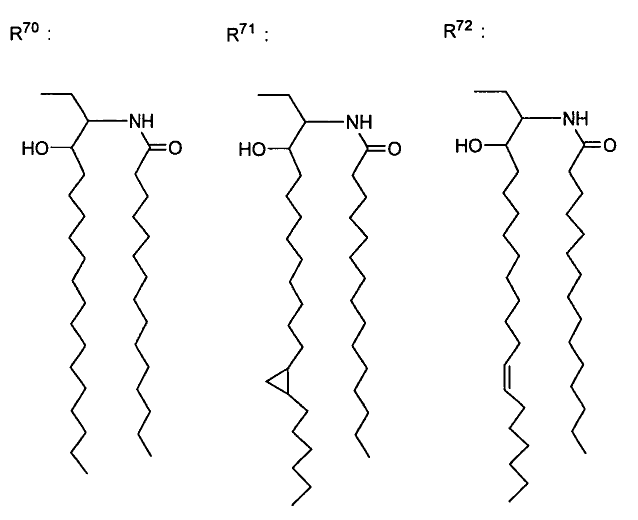

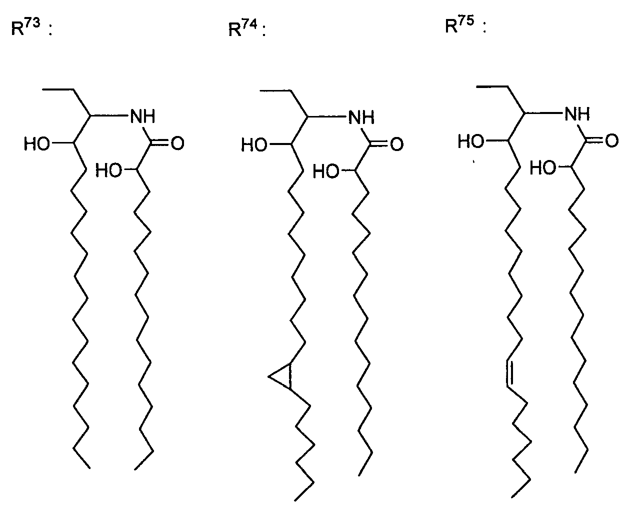

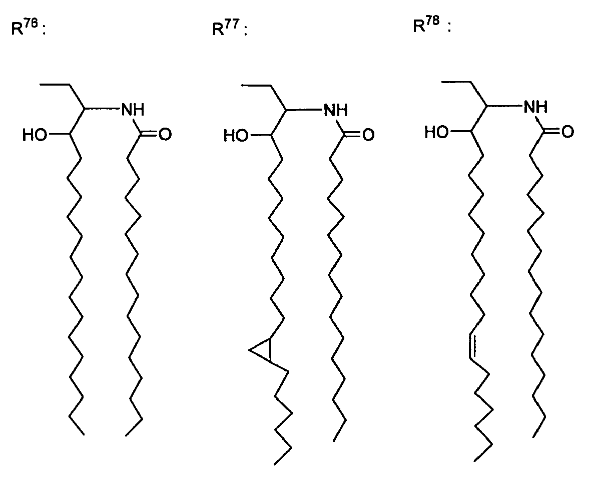

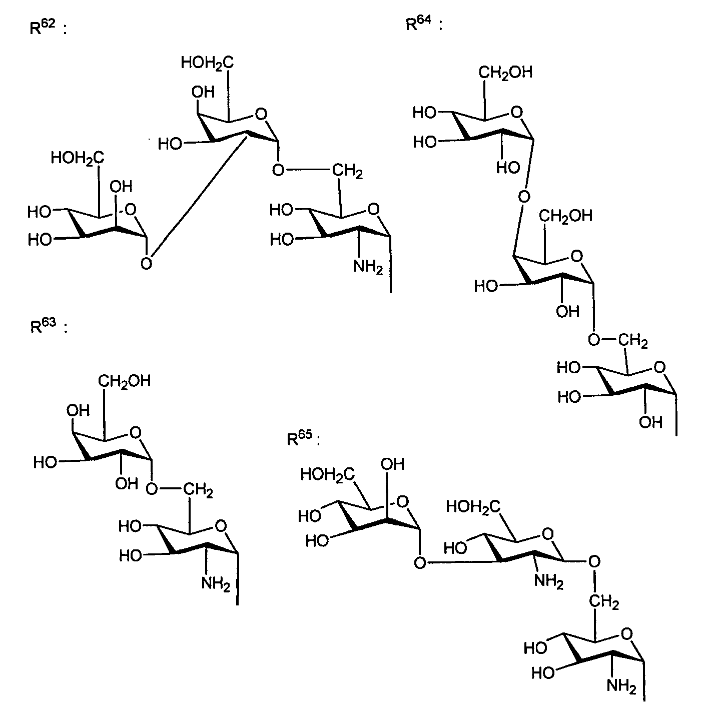

17. An antiallergic composition comprising a glycosphingolipid having a structure represented by the following formula (1): 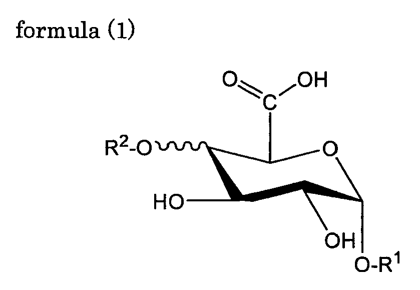 wherein R¹ represents the following formula (1-1): 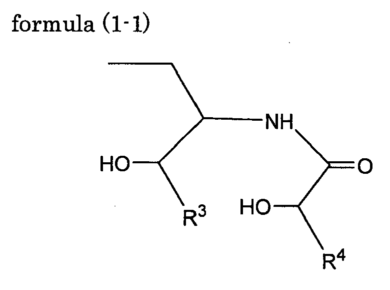 wherein R³ represents alkyl or alkenyl and R⁴ represents alkyl; and
R² represents hydrogen, or α-galactose, α-glucose, α-mannose, α-glucosamine, β-glucosamine or a combination thereof.

18. The antiallergic composition according to Claim 17, wherein the formula (1) is represented by the following formula (3): 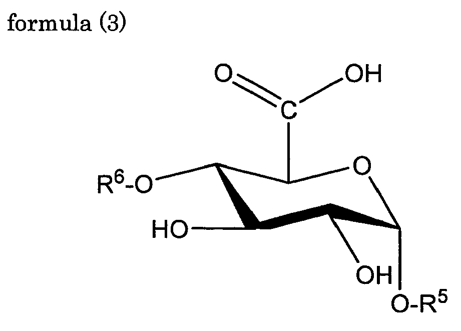 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

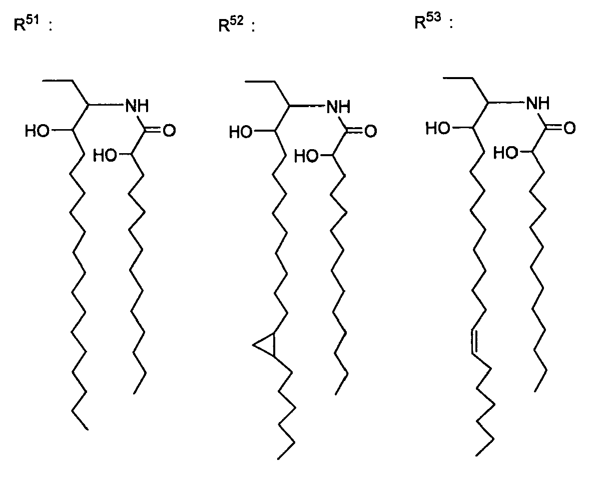

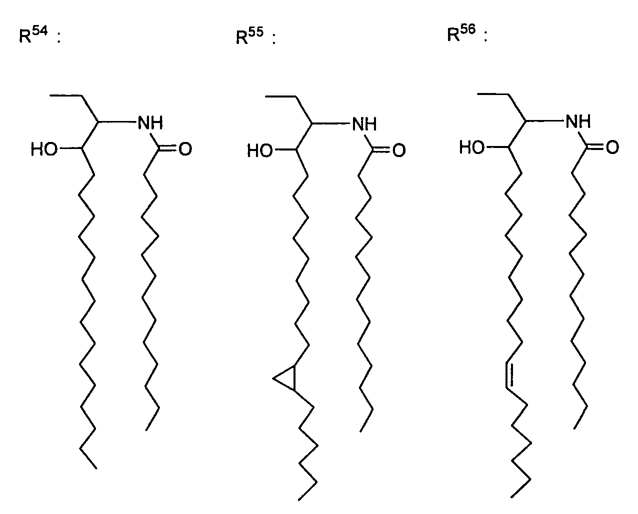

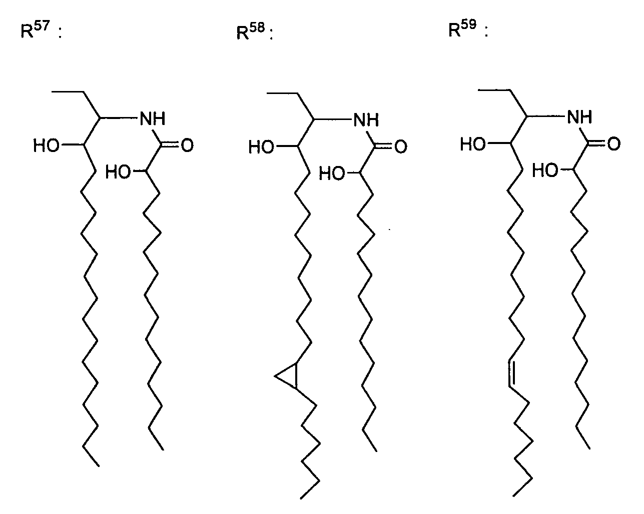

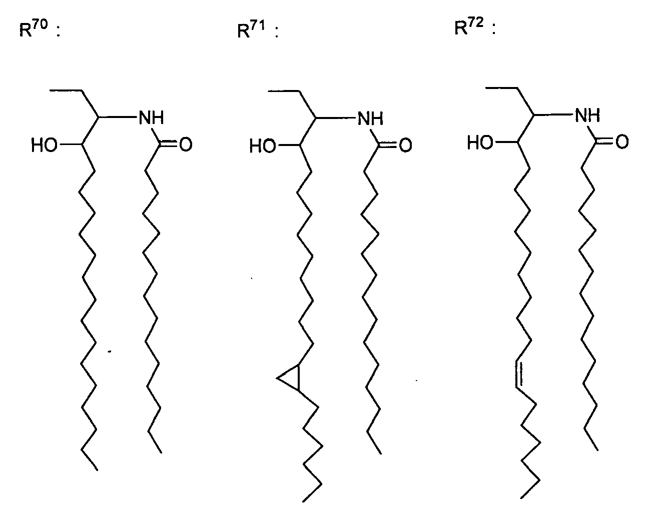

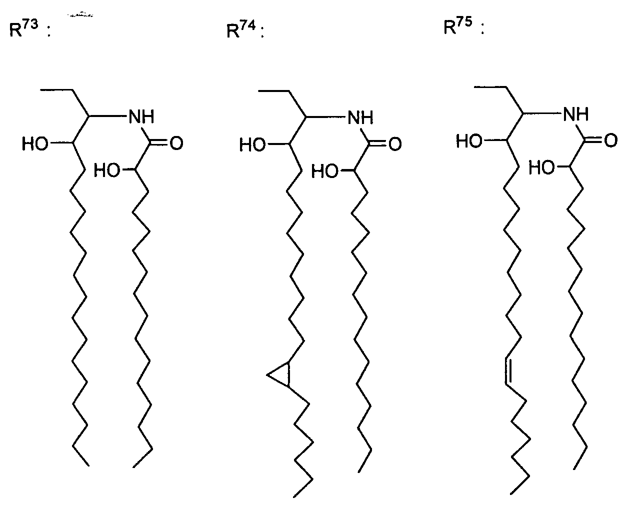

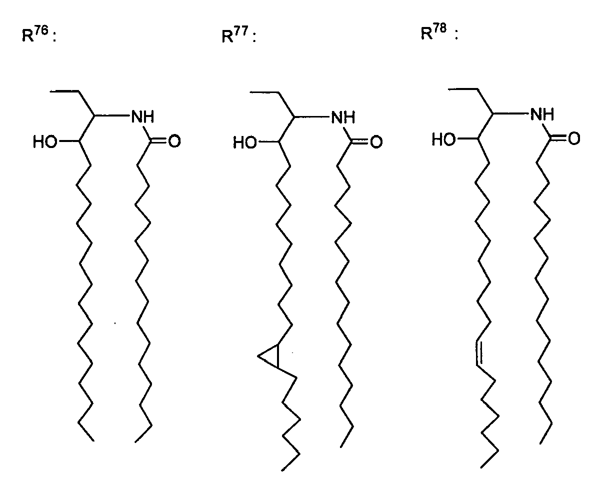

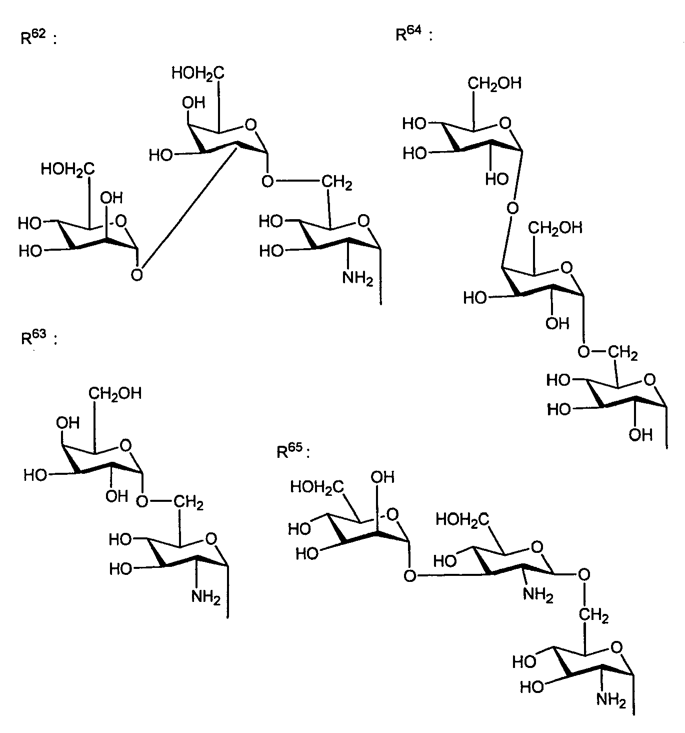

19. A composition for enhancing resistance to infection comprising a glycosphingolipid having a structure represented by the following formula (1): 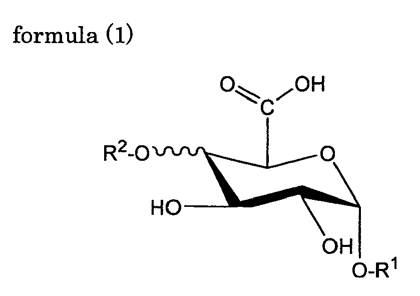 wherein R¹ represents the following formula (1-1): 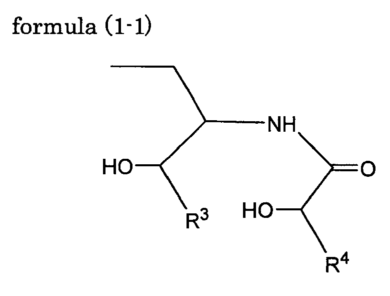 wherein R³ represents alkyl or alkenyl and R⁴ represents alkyl; and
R² represents hydrogen, or α-galactose, α-glucose, α-mannose, α-glucosamine, β-glucosamine or a combination thereof.

20. The composition for enhancing resistance to infection according to Claim 19, wherein the formula (1) is represented by the following formula (3): 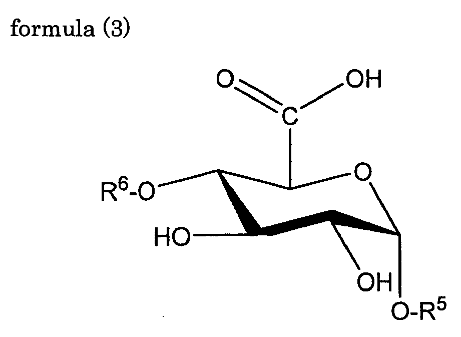 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

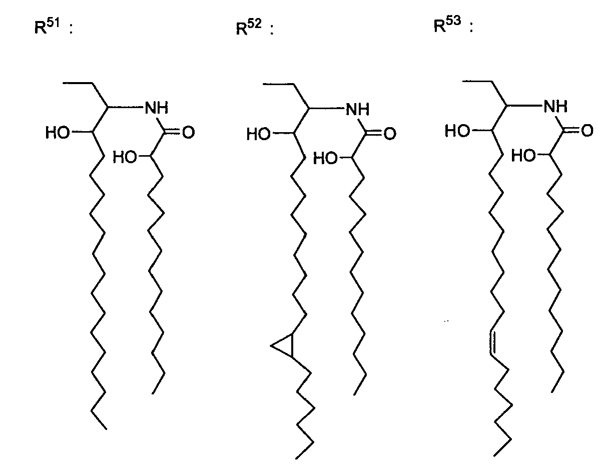

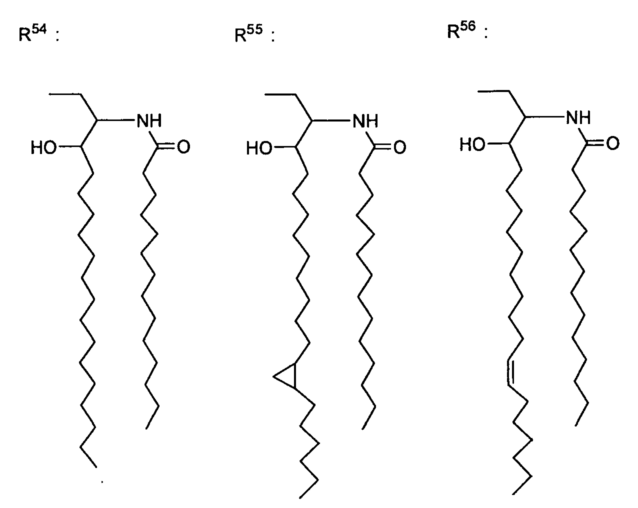

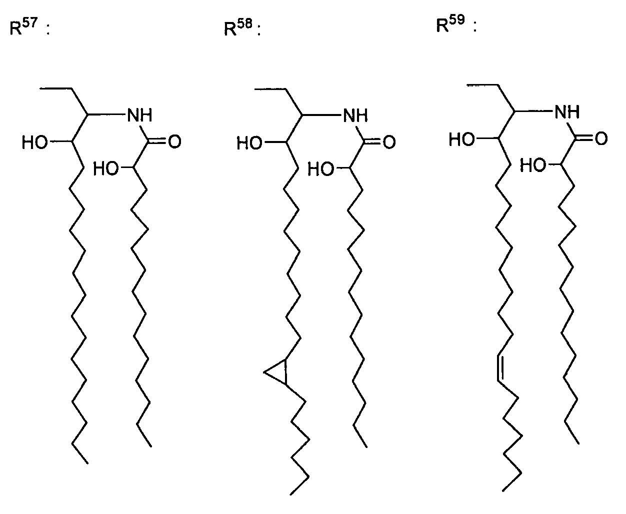

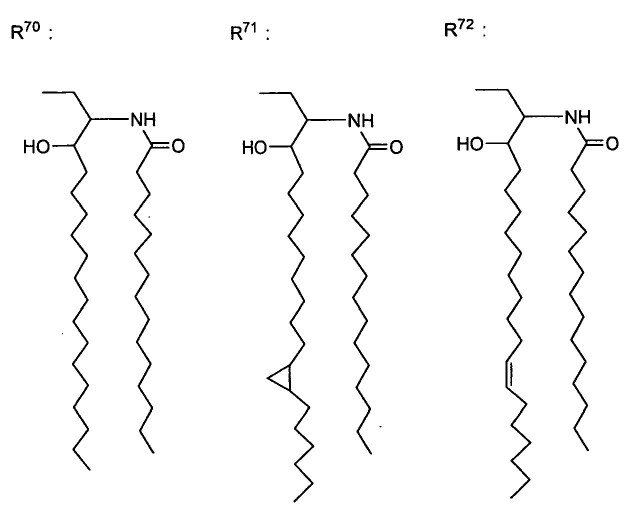

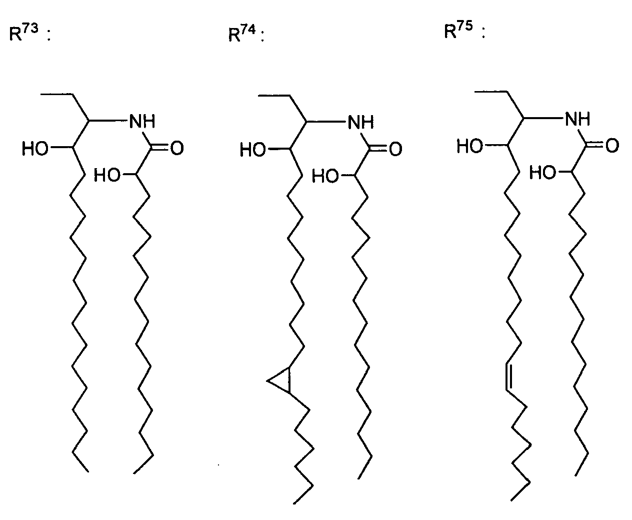

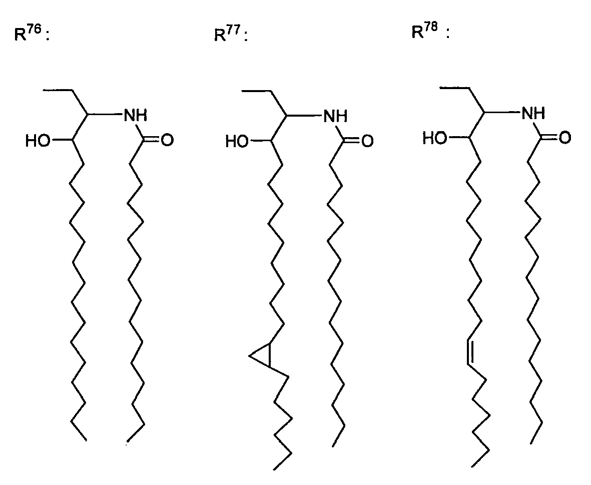

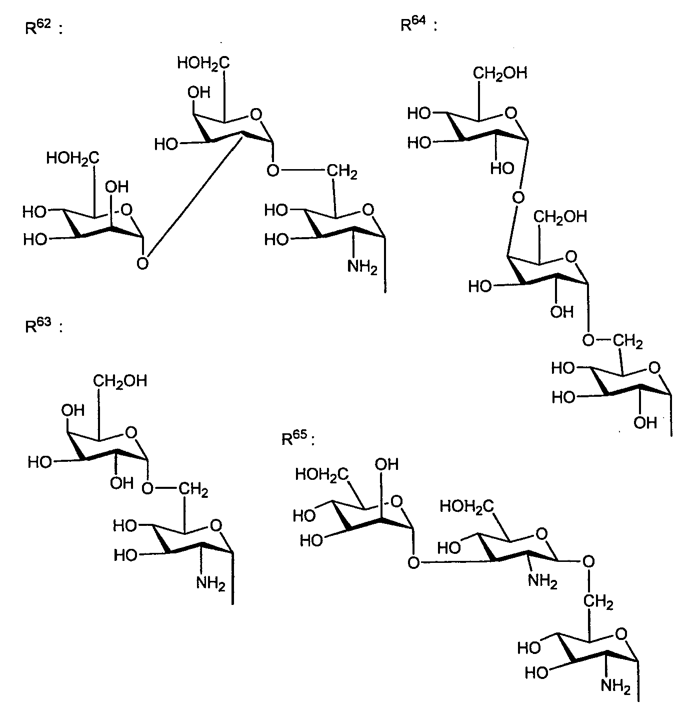

21. An antiviral composition comprising a glycosphingolipid having a structure represented by the following formula (1): 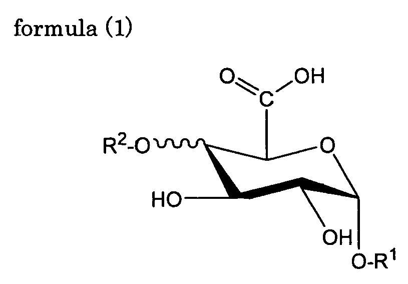 wherein R¹ represents the following formula (1-1): 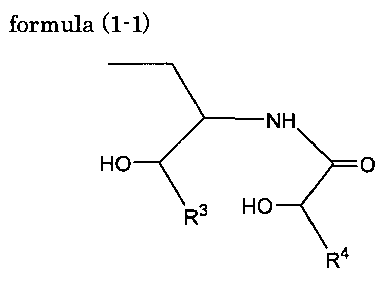 wherein R³ represents alkyl or alkenyl and R⁴ represents alkyl; and
R² represents hydrogen, or α-galactose, α-glucose, α-mannose, α-glucosamine, β-glucosamine or a combination thereof.

22. The antiviral composition according to Claim 21, wherein the formula (1) is represented by the following formula (3): 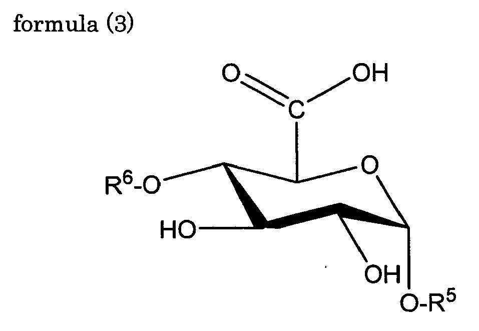 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

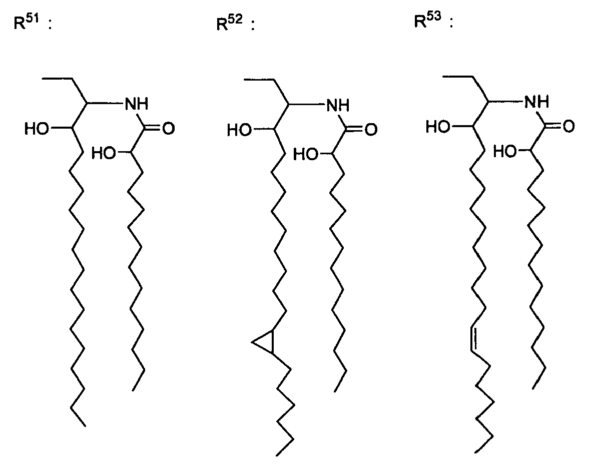

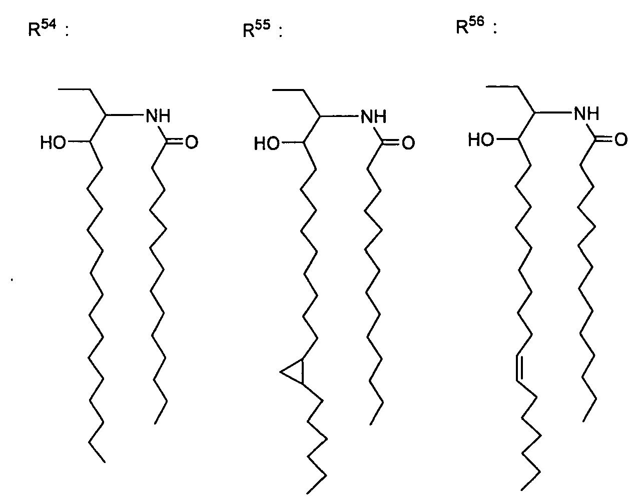

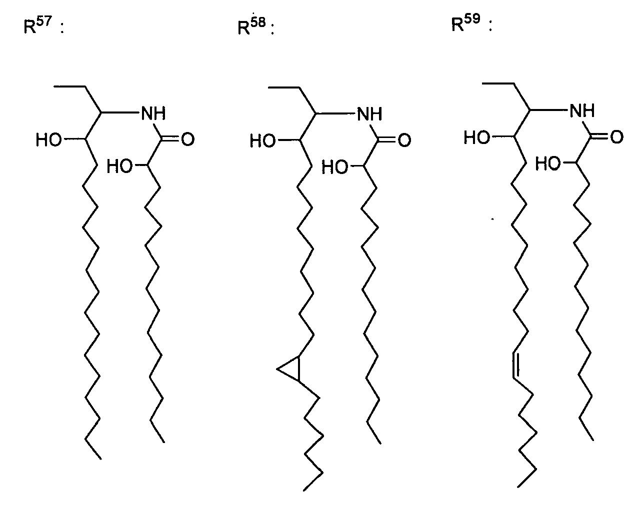

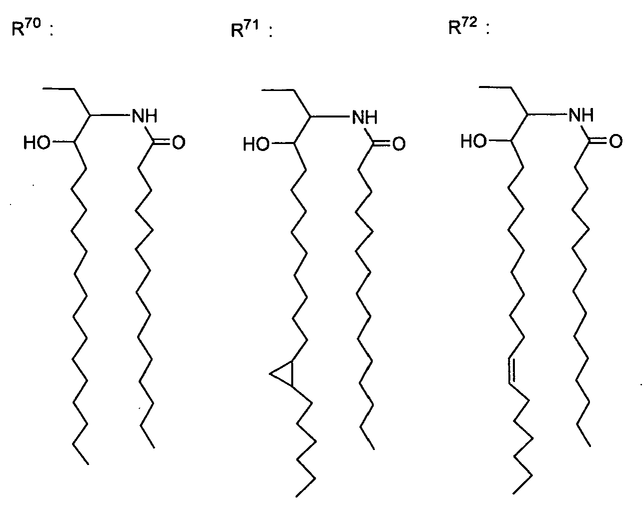

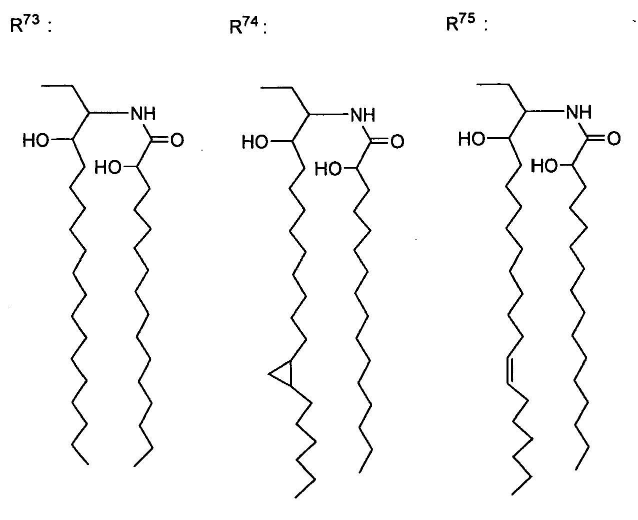

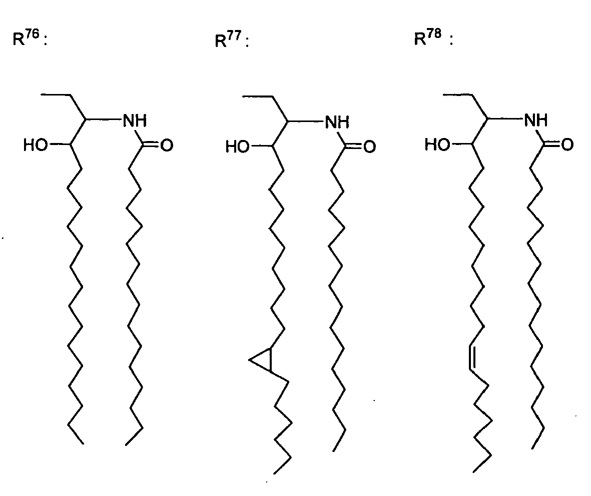

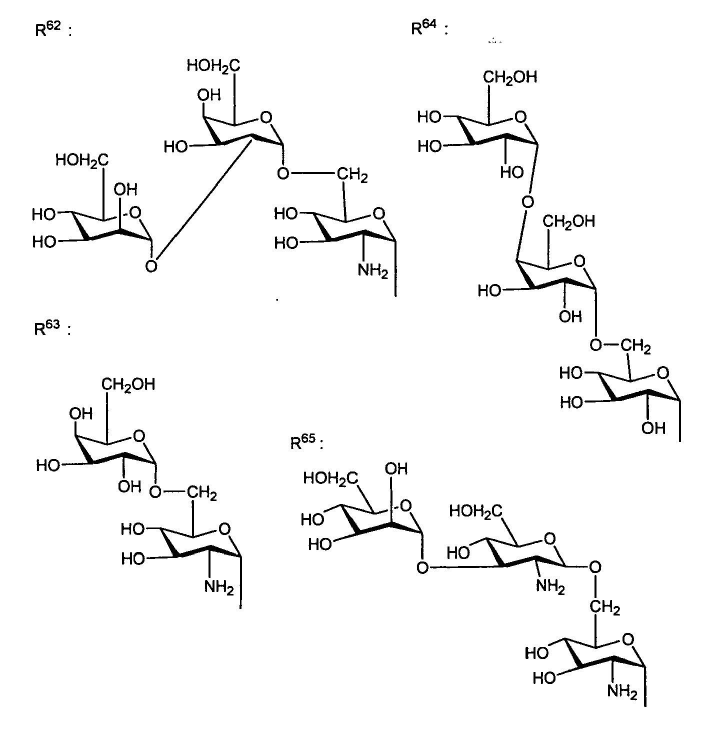

23. A composition for accelerating IL-6 production comprising a glycosphingolipid having a structure represented by the following formula (1): 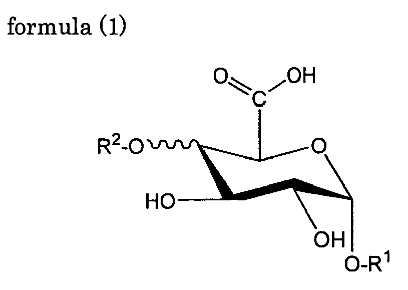 wherein R¹ represents the following formula (1-1): 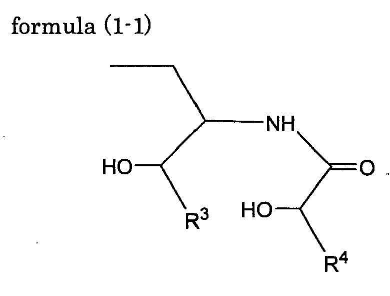 wherein R³ represents alkyl or alkenyl and R⁴ represents alkyl; and
R² represents hydrogen, or α-galactose, α-glucose, α-mannose, α-glucosamine, β-glucosamine or a combination thereof.

24. The composition for accelerating IL-6 production according to Claim 23, wherein the formula (1) is represented by the following formula (3): 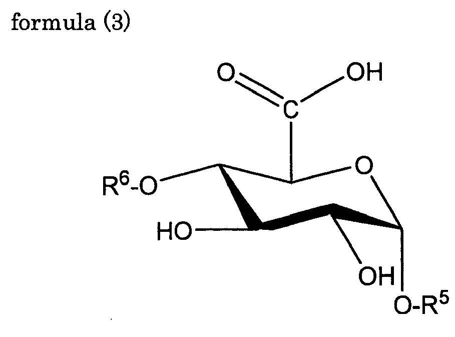 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:

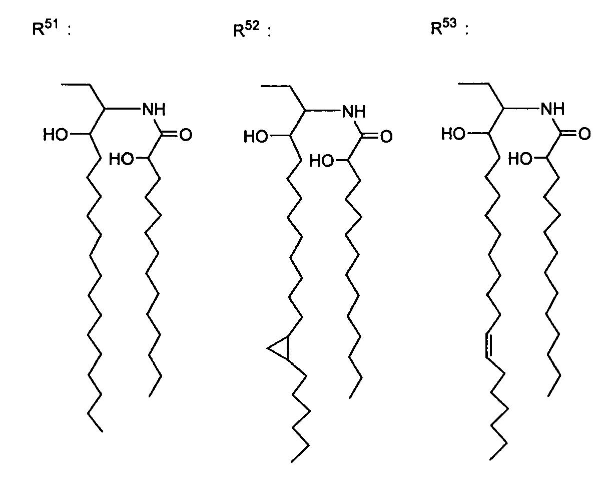

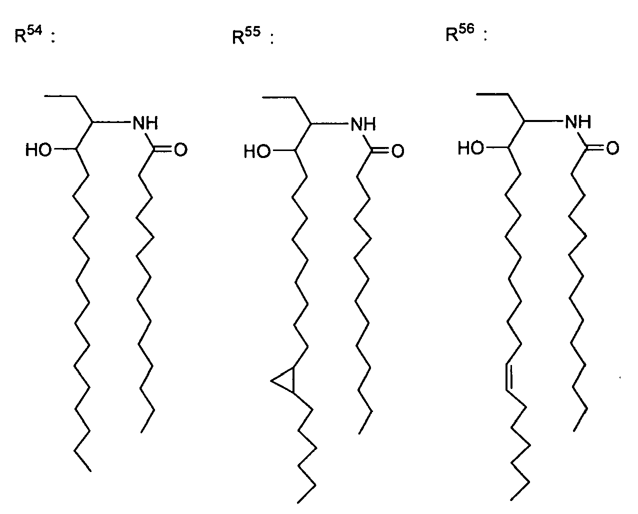

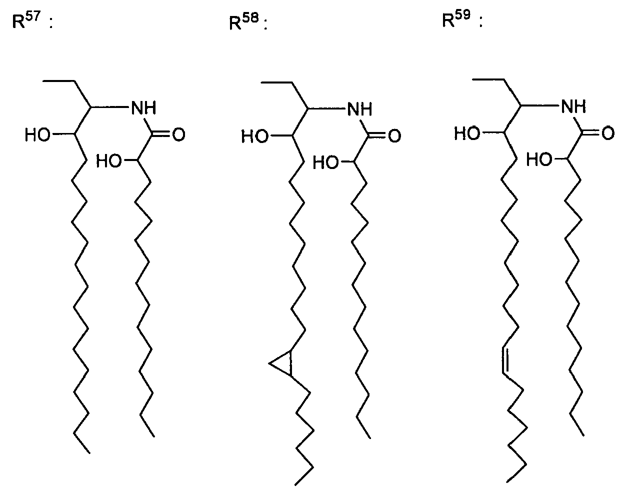

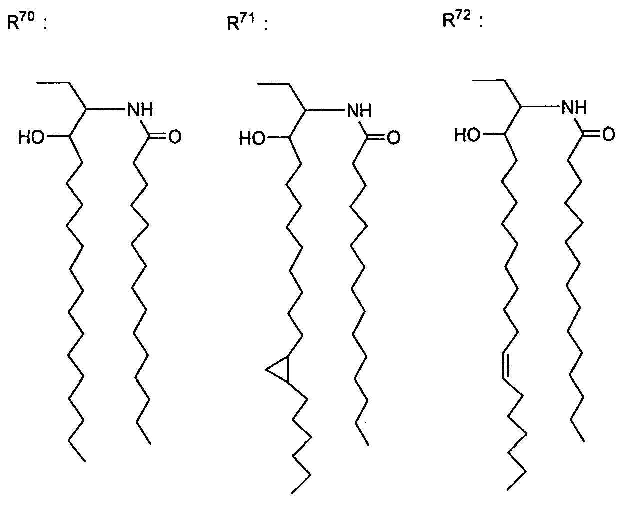

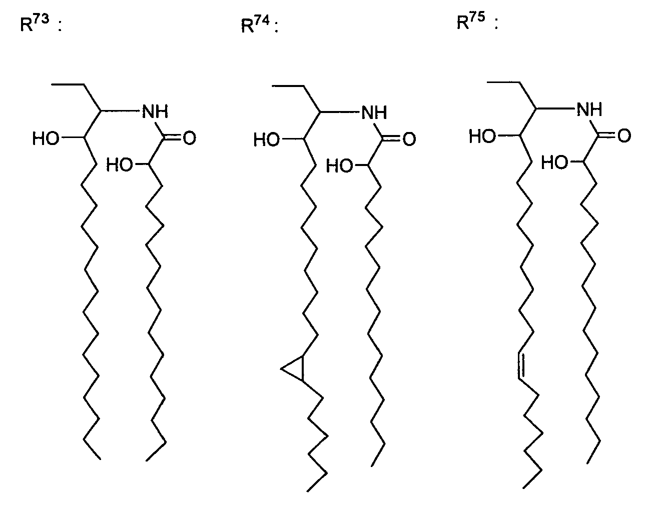

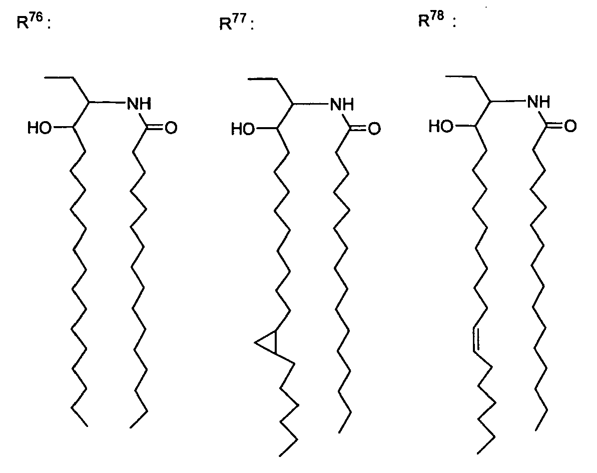

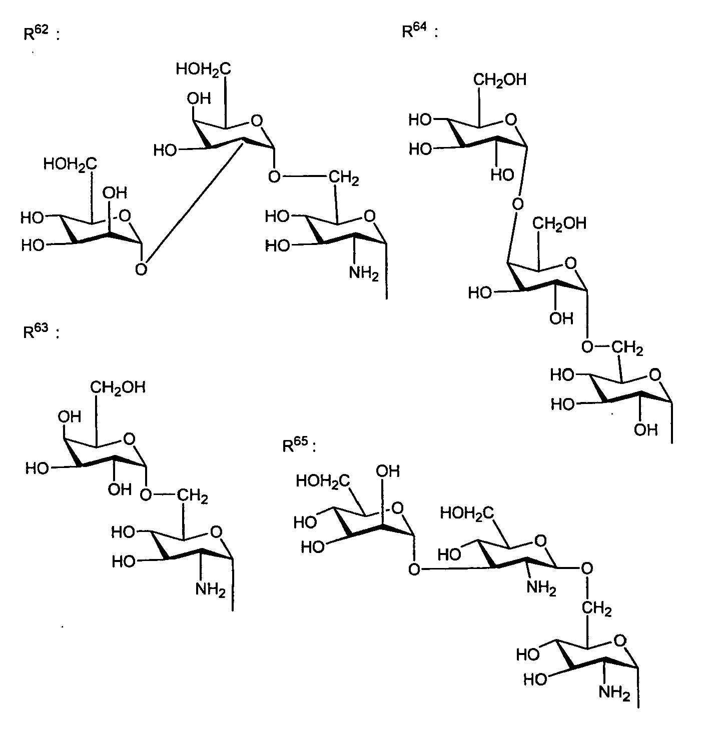

25. A composition for accelerating NO production comprising a glycosphingolipid having a structure represented by the following formula (1): 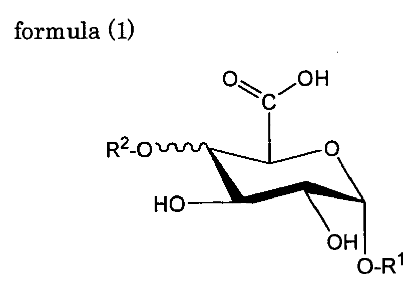 wherein R¹ represents the following formula (1-1): 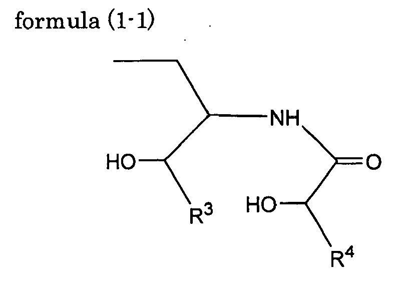 wherein R³ represents alkyl or alkenyl and R⁴ represents alkyl; and
R² represents hydrogen, or α-galactose, α-glucose, α-mannose, α-glucosamine, β-glucosamine or a combination thereof.

26. The composition for accelerating NO production according to Claim 25, wherein the formula (1) is represented by the following formula (3): 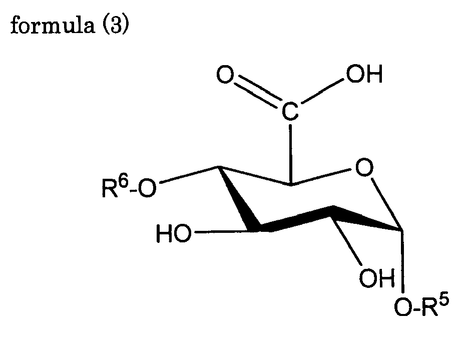 wherein R⁵ represents R⁵¹, R⁵², R⁵³, R⁵⁴, R⁵⁵, R⁵⁶, R⁵⁷, R⁵⁸, R⁵⁹, R⁷⁰, R⁷¹, R⁷², R⁷³, R⁷⁴, R⁷⁵, R⁷⁶, R⁷⁷, or R⁷⁸; and R⁶ represents hydrogen, R⁶², R⁶³, R⁶⁴, or R⁶⁵:
